(19) Europäisches Patentamt
European Patent Office
Office européen des brevets

(11) EP 1 849 453 B2

(12) # NOUVEAU FASCICULE DE BREVET EUROPEEN

Après la procédure d'opposition

(45) Date de publication et mention de la décision concernant l'opposition:
**27.05.2020 Bulletin 2020/22**

(45) Mention de la délivrance du brevet:
**01.06.2016 Bulletin 2016/22**

(21) Numéro de dépôt: **07106722.7**

(22) Date de dépôt: **23.04.2007**

(51) Int Cl.:
***A61K 8/06*** *(2006.01)* ***A61K 8/37*** *(2006.01)*
***A61K 8/895*** *(2006.01)* ***A61Q 5/02*** *(2006.01)*
***A61Q 5/12*** *(2006.01)*

(54) **Composition de lavage des matières kératiniques et procédé de traitement cosmétique mettant en oeuvre ladite composition**

Zusammensetzung zum Waschen von keratinhaltigen Stoffen und kosmetisches Behandlungsverfahren, bei dem diese Zusammensetzung eingesetzt wird.

Composition for washing keratinous material and cosmetic treatment method using said composition.

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **28.04.2006 FR 0603848**

(43) Date de publication de la demande:
**31.10.2007 Bulletin 2007/44**

(73) Titulaire: **L'Oréal**
**75008 Paris (FR)**

(72) Inventeurs:
- **Decoster, Sandrine**
  **95210, SAINT-GRATIEN (FR)**
- **Masse, Virginie**
  **92110, CLICHY (FR)**
- **Lesch, Sandie**
  **75013, PARIS (FR)**

(74) Mandataire: **Dossmann, Gérard**
**Casalonga & Partners**
**Bayerstrasse 71-73**
**80335 München (DE)**

(56) Documents cités:

| | |
|---|---|
| **EP-A- 0 453 238** | **EP-A- 0 697 206** |
| **EP-A- 0 870 491** | **EP-A- 0 974 335** |
| **WO-A-95/26707** | **DE-A1- 19 518 449** |
| **FR-A- 2 785 181** | **FR-A- 2 804 020** |
| **FR-A- 2 864 767** | **US-A- 3 950 417** |
| **US-A- 6 153 569** | **US-A1- 2001 009 909** |

- **Essais comparatifs soumis le 10.11.2008 pendant la procédure d' examen**
- **Becher, P: "Dictionary of Colloid and Surface Science", 1990 pages 100-103,**
- **Bourrel, M. et Schecjter, R.: "Microemulsions and Related Systems", 1988 pages 24-27,**
- **Le Hir, A.: "l? Abrégé de Pharmacie Galénique", 1981 pages 132-133,**
- **Essais comparatifs, deuxieme série, soumis le 26.10.2010 pendant la procédure d'examen**
- **Essais comparatifs fournis par la titulaire datés du 05.02.2018**
- **Fiche technique du mélange 1-(hexadécyloxy)-2-octadécanol et 1-hexadécanol**
- **Tests comparatifs fournis par l'opposant daté du 04.04.2018**

Remarques:
Le dossier contient des informations techniques présentées postérieurement au dépôt de la demande et ne figurant pas dans le présent fascicule.

EP 1 849 453 B2

## Description

La présente invention est relative à une composition cosmétique améliorée pour le lavage des matières kératiniques, en particulier des cheveux et/ou de la peau, et qui se présente sous la forme d'une microémulsion à base d'eau et d'au moins une huile choisie parmi les silicones aminées.

La présente invention concerne également un procédé de traitement cosmétique mettant en oeuvre une telle composition.

De nombreuses compositions de lavage des matières kératiniques ont été décrites dans l'art antérieur.

Ainsi, la demande de brevet FR 2 804 020 décrit des compositions de lavage, notamment des shampooings, comprenant au moins un tensioactif détergent et au moins un ester d'acide gras et de sorbitan oxyéthyléné ayant un nombre de moles d'oxyéthylène inférieur ou égal à 10.

Le brevet US 6 153 569 décrit des compositions de shampooings transparentes présentant à la fois de bonnes propriétés d'usage (bon pouvoir moussant, facilité d'application) et un pouvoir conditionnant amélioré. Ces compositions comprennent une microémulsion de silicone aminée, au moins un tensioactif anionique détergent, au moins un booster de mousse, au moins un agent d'ajustement du pH, au moins un agent épaississant et de l'eau.

La demande de brevet EP 453 238 décrit des compositions de shampooings douces dont le pouvoir moussant est amélioré. Ces compositions comprennent, en milieu aqueux, de 8 à 25 % en poids d'un mélange de tensioactifs constitué de :

- un tensioactif anionique,
- un tensioactif amphotère, à l'exception des bétaïnes contenant du phosphore,
- un tensioactif non ionique oxyalkylé ou glycosidique ayant une HLB d'au moins 8.

La demande de brevet WO 02/05758 décrit une composition de nettoyage automoussante, dont le pouvoir moussant et la facilité d'application sont améliorés. Cette composition comprend au moins un agent automoussant, associé à un mélange de tensioactifs comprenant au moins un tensioactif anionique, au moins un tensioactif amphotère, et, en option, au moins un tensioactif non ionique. Cette composition peut en outre comprendre un ou plusieurs agents conditionneurs cationiques tels que par exemple des dérivés cationiques de cellulose, des dérivés cationiques de guar, des dérivés et copolymères de chlorure de diallyldiméthylammonium.

Le brevet CA 1 077 849 décrit des compositions détergentes et des shampooings diminuant les risques d'irritation oculaire et dont le pouvoir moussant (volume et stabilité de la mousse) est amélioré. Ces compositions comprennent une bétaïne tensioactive particulière, un tensioactif anionique, et un tensioactif non-ionique constitué d'un dérivé polyoxyéthyléné, soluble dans l'eau, d'une base hydrophobe, avec un rapport molaire entre la quantité de bétaïne et la quantité de tensioactif anionique comprise entre 0,9 / 1 et 1,1 / 1.

La demande de brevet WO 03/057185 décrit des compositions cosmétiques ou dermatologiques pour le soin ou le nettoyage de la peau et des cheveux, qui permettent de combiner douceur et bon pouvoir nettoyant. Ces compositions sont basées sur une combinaison synergique de tensioactifs anioniques et non ioniques comprenant des alkylpolyglycosides, des alkykl(éther)sulfates, des esters de sorbitan polyéthylèneglycol et des alkyl citrate sulfosuccinates.

Toutefois, les compositions décrites dans l'art antérieur présentent certaines insuffisances. En particulier, les shampooings les plus performants peuvent provoquer des picotements dans l'œil lorsque le produit dilué coule dans la sphère oculaire, ce qui arrive fréquemment chez les enfants. Par ailleurs, bon nombre de ces compositions provoquent, chez les personnes présentant une peau sensible, des réactions d'inconfort telles que des rougeurs, des démangeaisons, des picotements.

Les compositions douces proposées dans l'art antérieur présentent à l'inverse des propriétés cosmétiques insuffisantes, notamment en termes de douceur et, en ce qui concerne les cheveux, de démêlage, de lissage et de légèreté.

La Demanderesse a maintenant découvert de manière surprenante qu'en utilisant des microémulsions particulières comprenant certaines huiles (silicones aminées) et une combinaison particulière de tensioactifs, il était possible de formuler des compositions de lavage particulièrement douces, présentant en outre d'excellentes propriétés cosmétiques.

Ainsi, les compositions selon l'invention permettent de diminuer les réactions d'inconfort au niveau de la peau et du cuir chevelu, et possèdent un excellent niveau de tolérance oculaire. Parallèlement, elles présentent de très bonnes qualités d'usage, et d'excellentes propriétés cosmétiques, notamment en termes de douceur et, en ce qui concerne les cheveux outre la douceur, de démêlage, de lissage et de légèreté.

La présente invention a donc pour objet une composition cosmétique pour le lavage des matières kératiniques constituée d'une microémulsion comprenant :

- de l'eau,

- du mono-laurate de sorbitan oxyéthylène à 4 OE,
- au moins une huile choisie parmi les silicones aminées,
- au moins un tensioactif anionique, et
- au moins un tensioactif amphotère ou zwittérionique.

Un autre objet de l'invention est un procédé de traitement cosmétique mettant en œuvre ladite composition.

D'autres objets et caractéristiques, aspects et avantages de l'invention apparaîtront plus clairement à la lecture de la description et des exemples qui suivent.

La composition selon l'invention se présente sous la forme d'une microémulsion à base d'eau et d'huile.

Par microémulsion, on désigne, de manière connue en soi, non pas une émulsion véritable, mais une solution transparente, thermodynamiquement stable, de micelles gonflées par de l'huile. En d'autres termes, l'huile est solubilisée dans l'eau en présence d'un ou plusieurs tensioactifs. Le terme microémulsion est notamment défini dans le "Dictionary of Colloid and Surface Science" de Paul Becher, publié en 1990 par Marcel Dekker Inc, page 102.

De préférence, le diamètre moyen en nombre des particules d'huile, mesuré par granulométrie laser, est inférieur ou égale à 100 nm, de préférence inférieur ou égale à 50 nm, et mieux encore inférieur ou égale à 20 nm.

Ces microémulsions se distinguent des nanoémulsions, dans lesquelles les particules d'huile peuvent présenter des tailles initiales du même ordre de grandeur, mais qui sont thermodynamiquement instables, nécessitant pour leur préparation un apport d'énergie important, et qui sont susceptibles d'évoluer dans le temps.

Les microémulsions selon l'invention ont un aspect translucide ou transparent, et de préférence transparent.

La transparence peut se mesurer par mesure de la transmittance à 700nm via un spectromètre d'absorption dans le visible (par exemple spectromètre Lambda 14 de Perkin Elmer ou UV2101PC de Shimadzu). La mesure se fait sur la composition non diluée. Le blanc est fait avec de l'eau distillée.

Les compositions selon l'invention ont une transmittance de préférence supérieure ou égale à 85%, plus préférentiellement supérieure ou égale à 90 % et encore plus préférentiellement supérieure ou égale à 94%. De préférence, la transmittance est comprise entre 96 et 100%.

Les microémulsions conformes à la présente invention peuvent contenir des solvants notamment pour améliorer, si nécessaire, la transparence de la formulation.

Ces solvants sont choisis de préférence dans le groupe formé par :

- les alcools inférieurs en $C_1$-$C_8$ tels que l'éthanol ;
- les glycols tels que la glycérine, le propylèneglycol, le 1,3-butylèneglycol, le dipropylèneglycol, l'hexylèneglycol, les polyéthylèneglycols comportant de 4 à 16 unités d'oxyde d'éthylène et de préférence de 8 à 12.

La composition selon l'invention comprend au moins une huile choisie parmi les silicones aminées.

On désigne par silicone aminée toute silicone comportant au moins une amine primaire, secondaire, tertiaire ou un groupement ammonium quaternaire. On peut ainsi citer :

a) les polysiloxanes répondant à la formule (A):

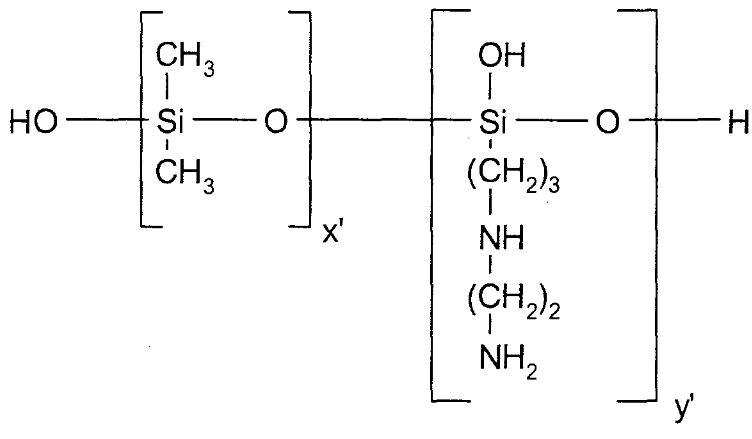
(A)

dans laquelle x' et y' sont des nombres entiers dépendant du poids moléculaire, généralement tels que ledit poids moléculaire moyen en poids est allant de 5 000 et 500 000 environ ;

b) les silicones aminées répondant à la formule :

$$R'_aG_{3-a}\text{-}Si(OSiG_2)_n\text{-}(OSiG_bR'_{2-b})_m\text{-}O\text{-}SiG_{3-a}\text{-}R'_a \qquad (B)$$

dans laquelle :

G, identiques ou différents, désignent un atome d'hydrogène, ou un groupement phényle, OH, alkyle en $C_1$-$C_8$, par exemple méthyle, ou alcoxy en $C_1$-$C_8$, par exemple méthoxy,
a identiques ou différents, désignent le nombre 0 ou un nombre entier de 1 à 3, en particulier 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;

R', identiques ou différents, désignent un radical monovalent de formule -CqH2qL dans laquelle q est un nombre de 2 à 8 et L est un groupement aminé éventuellement quaternisé choisi parmi les groupements :

- NR''-Q-N(R'')$_2$
- N(R'')$_2$
- N$^+$(R'')$_3$ A-
- N$^+$H(R'')$_2$ A-
- N$^+$H$_2$(R'') A-
- N(R'')-Q-N$^+$R''H$_2$ A-
- NR''-Q-N$^+$ (R'')$_2$H A-
- NR''-Q-N$^+$ (R'')$_3$ A-,

dans lesquels R'' peut désigner hydrogène, phényle, benzyle, ou un radical hydrocarboné saturé monovalent, par exemple un radical alkyle ayant de 1 à 20 atomes de carbone ; Q désigne un groupement de formule $C_rH_{2r}$, linéaire ou ramifié, r étant un entier allant de 2 à 6, de préférence de 2 à 4 ; et A- représente un ion halogénure tel que par exemple fluorure, chlorure, bromure ou iodure.

Un groupe de silicones aminées correspondant à cette définition est représentée par les silicones dénommées "triméthylsilylamodiméthicone", répondant à la formule :

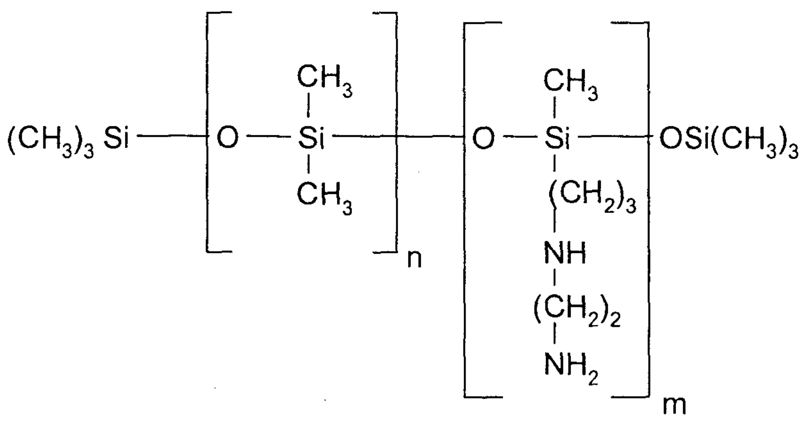

(C)

dans laquelle n et m ont les significations données ci-dessus (cf formule B).

De tels polymères sont décrits par exemple dans la demande de brevet EP-A-95238.

Un autre groupe de silicones aminées correspondant à cette définition est représentée par les silicones de formules (D) ou (E) suivantes :

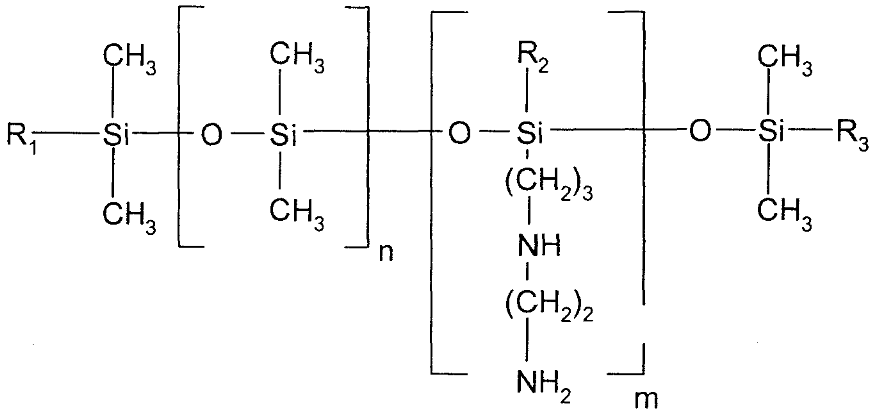

(D)

dans laquelle :

m et n sont des nombres tels que la somme (n + m) peut varier notamment de 1 à 1 000 et en particulier de 50 à 250 et plus particulièrement de 100 à 200,
n pouvant désigner un nombre de 0 à 999 et notamment de 49 à 249 et plus particulièrement de 125 à 175 et m pouvant désigner un nombre de 1 à 1 000, et notamment de 1 à 10 et plus particulièrement de 1 à 5,
$R_1$, $R_2$, $R_3$, identiques ou différents, représentent un radical hydroxy ou alcoxy en $C_1$-$C_4$, l'un au moins des radicaux $R_1$ à $R_3$ désignant un radical alcoxy.

De préférence le radical alcoxy est un radical méthoxy.

Le rapport molaire hydroxy/alcoxy va de préférence de 0,2:1 à 0,4:1 et de préférence de 0,25:1 à 0,35:1 et plus particulièrement est égal à 0,3:1.

La masse moléculaire moyenne en poids de la silicone va de préférence de 2000 à 1000000 et encore plus particulièrement de 3500 à 200000.

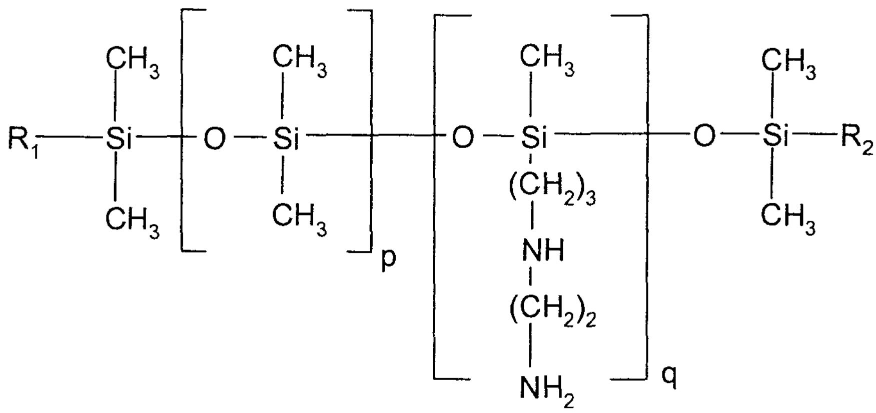

(E)

dans laquelle :

p et q sont des nombres tels que la somme (p+q) peut varier notamment de 1 à 1 000 et en particulier de 50 à 350, et plus particulièrement de 150 à 250,
p pouvant désigner un nombre de 0 à 999 et notamment de 49 à 349 et plus particulièrement de 159 à 239 et q pouvant désigner un nombre de 1 à 1 000, et notamment de 1 à 10 et plus particulièrement de 1 à 5;
$R_1$, $R_2$, différents, représentent un radical hydroxy ou alcoxy en $C_1$-$C_4$, l'un au moins des radicaux $R_1$ ou $R_2$ désignant un radical alcoxy.

De préférence le radical alcoxy est un radical méthoxy.

Le rapport molaire hydroxy/alcoxy va généralement de 1:0,8 à 1:1,1 et de préférence de 1:0,9 à 1:1 et plus particulièrement est égal à 1:0,95.

La masse moléculaire moyenne en poids de la silicone va de préférence de 2000 à 200000 et encore plus particulièrement de 5000 à 100000 et plus particulièrement de 10000 à 50000.

Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie.

Les produits commerciaux correspondant à ces silicones de structure (D) ou (E) peuvent inclure dans leur composition une ou plusieurs autres silicones aminées dont la structure est différente des formules (D) ou (E).

Un produit contenant des silicones aminées de structure (D) est proposé par la société WACKER sous la dénomination BELSIL® ADM 652.

Un produit contenant des silicones aminées de structure (E) est proposé par WACKER sous la dénomination Fluid WR 1300®.

Lorsque ces silicones aminées sont mises en œuvre, une forme de réalisation particulièrement intéressante est leur utilisation sous forme d'émulsion huile dans eau. L'émulsion huile dans eau peut comprendre un ou plusieurs tensioactifs.

Les tensioactifs peuvent être de toute nature mais de préférence cationique et/ou non ionique.

La taille moyenne en nombre des particules de silicone dans l'émulsion va généralement de 3 nm à 500 nanomètres .

De préférence, notamment comme silicones aminées de formule (E), on utilise des microémulsions dont la taille moyenne des particule va de 5 nm à 60 nanomètres (bornes incluses) et plus particulièrement de 10 nm à 50 nanomètres (bornes incluses).

Ainsi, on peut utiliser selon l'invention les microémulsions de silicone aminée de formule (E) proposées sous les dénominations FINISH CT 96 E® ou SLM 28020® par la société WACKER.

De préférence la silicone aminée est choisie de façon telle que l'angle de contact avec l'eau d'un cheveu traité avec une composition contenant 2% (matière active) de ladite silicone selon l'invention varie de 90° à 180° (bornes incluses) et de préférence de 90 à 130° (bornes incluses).

Pour mesurer l'angle de contact, la silicone aminée est de préférence solubilisée ou dispersée dans un solvant de la silicone aminée ou de l'émulsion de silicone aminée (notamment l'hexaméthyldisiloxane ou l'eau selon l'hydrophilie de la silicone).

De préférence la composition contenant la ou les silicones aminées de formule (D) ou (E) est telle que l'angle de contact avec l'eau d'un cheveu traité avec ladite composition varie de 90 à 180°(bornes incluses) et de préférence de 90 à 130° (bornes incluses).

La mesure de l'angle de contact est basée sur l'immersion d'un cheveu dans de l'eau distillée. Il consiste à évaluer la force exercée par l'eau sur le cheveu lors de son immersion de l'eau distillée et lors de son retrait. Les forces ainsi mesurées sont directement reliées à l'angle de contact $\theta$ entre l'eau et la surface du cheveu. Le cheveu est dit hydrophile lorsque l'angle $\theta$ est situé de 0 à 90° (bornes incluses), hydrophobe lorsque cet angle est situé de 90° à 180° (bornes incluses).

Le test s'effectue avec des mèches de cheveux naturels ayant été décolorés dans les mêmes conditions puis lavés.

Chaque mèche de 1 g est placée dans un cristallisoir de 75 mm de diamètre puis recouverte de façon homogène par 5 mL de la formule à tester. La mèche est ainsi laissée 15 minutes à température ambiante puis rincée à l'eau distillée pendant 30 secondes. La mèche essorée est laissée à l'air libre jusqu'à ce qu'elle soit complètement sèche.

Pour chaque évaluation, 10 cheveux ayant subi le même traitement sont analysés. Chaque échantillon, fixé à une microbalance de précision, est immergé par la pointe dans un récipient rempli d'eau distillée. Cette balance DCA (« Dynamic Contact Angle Analyser »), de la société CAHN Instruments, permet de mesurer la force (f) exercée par l'eau sur le cheveu.

Parallèlement, le périmètre du cheveu (p) est mesuré *via* une observation microscopique.

La force moyenne de mouillabilité sur 10 cheveux et la section des cheveux analysés permettent d'obtenir l'angle de contact du cheveu sur l'eau, selon la formule :

$$f = p * \Gamma lv * \cos\theta$$

où f est la force de mouillabilité exprimée en Newton, p le périmètre du cheveu en mètre, $\Gamma lv$ la tension interfaciale liquide / vapeur de l'eau en $J/m^2$ et $\theta$ l'angle de contact.

Le produit SLM 28020 de WACKER à 12% dans l'eau (soit 2% en silicone aminée) conduit à un angle de contact de 93° selon le test indiqué ci-dessus.

Le produit BELSIL ADM 652 de WACKER à 2% dans l'hexaméthyldisiloxane (soit 2% en silicone aminée)

conduit à un angle de contact de 111° selon le test indiqué ci-dessus.

Un autre groupe de silicones aminées correspondant à cette définition est représenté par la formule suivante (F):

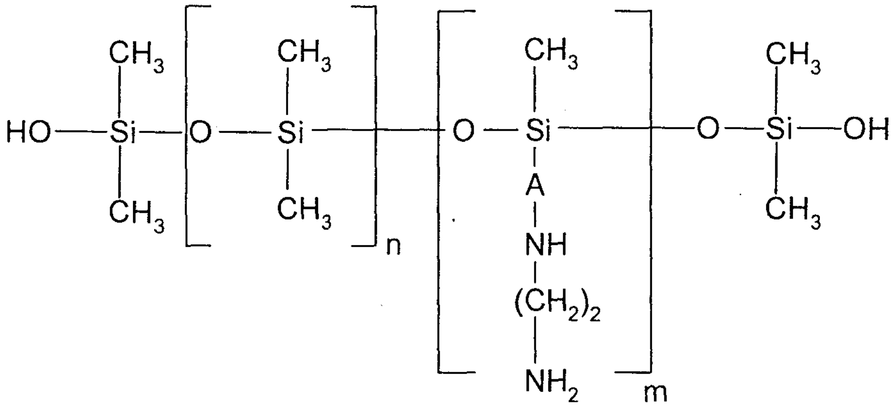

(F)

dans laquelle :

m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;

A désigne un radical alkylène linéaire ou ramifié ayant de 4 à 8 atomes de carbone et de préférence 4 atomes de carbone. Ce radical est de préférence linéaire.

La masse moléculaire moyenne en poids de ces silicones aminées va de préférence de 2000 à 1000000 et encore plus particulièrement de 3500 à 200.000.

Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie.

Selon l'invention la viscosité à 25°C de la silicone aminée est notamment supérieure à 25000 cSt ($mm^2/s$) et de préférence va de 30000 à 200000 cSt ($mm^2/s$). et plus particulièrement de 30000 à 150000 cSt ($mm^2/s$).

Ces silicones aminées, ont de préférence un indice d'amine inférieur ou égal à 0,4 meq./g, de préférence allant de 0,001 à 0,2 meq./g, et plus particulièrement allant de 0,01 à 0,1 meq./g.

L'indice d'amine est le nombre de milli-équivalents amine par gramme de composé. Cet indice est déterminé de façon tout à fait classique par des méthodes de titrage par indicateur coloré ou par titrage potentiométrique.

Lorsque ces silicones aminées sont mises en œuvre, une forme de réalisation particulièrement intéressante est leur utilisation sous forme d'émulsion huile dans eau. L'émulsion huile dans eau peut comprendre un ou plusieurs tensioactifs.

Les tensioactifs peuvent être de toute nature mais de préférence cationiques et/ou non ioniques.

La taille moyenne en nombre des particules de silicone dans l'émulsion va généralement de 3 nm à 500 nanomètres, de préférence de 5 nm à 300 nanomètres et plus particulièrement de 10 nm à 275 nanomètres et encore plus préférentiellement de 150 à 275 nanomètres.

Une silicone répondant à cette formule est par exemple la DC2-8299 CATIONIC EMULSION de DOW CORNING.

Un autre groupe de silicones aminées correspondant à cette définition est représenté par la formule suivante (G):

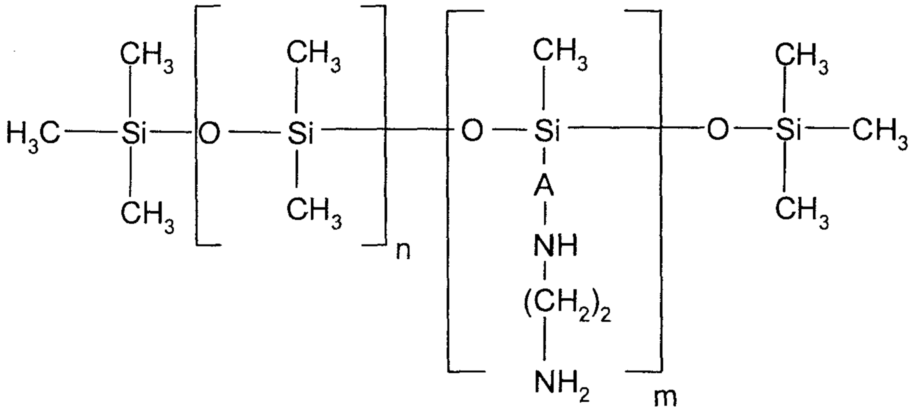

(G)

dans laquelle :

m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
A désigne un radical alkylène linéaire ou ramifié ayant de 4 à 8 atomes de carbone et de préférence 4 atomes de carbone. Ce radical est de préférence ramifié.

La masse moléculaire moyenne en poids de ces silicones aminées va de préférence de 500 à 1000000 et encore plus particulièrement de 1000 à 200.000.

Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie.

Selon l'invention la viscosité à 25°C de ces silicones aminées est notamment supérieure à 500 cSt ($mm^2/s$) et de préférence va de 1000 à 200000 cSt ($mm^2/s$). et plus particulièrement de 1500 à 10000 cSt ($mm^2/s$).

Ces silicones aminées, ont de préférence un indice d'amine supérieur à 0,4 meq./g, de préférence allant de 0,5 à 3 meq./g, et plus particulièrement allant de 0,5 à 1 meq./g.

L'indice d'amine est le nombre de milli-équivalents amine par gramme de composé. Cet indice est déterminé de façon tout à fait classique par des méthodes de titrage par indicateur coloré ou par titrage potentiométrique.

Lorsque ces silicones aminées sont mises en œuvre, une forme de réalisation particulièrement intéressante est leur utilisation sous forme d'émulsion huile dans eau. L'émulsion huile dans eau peut comprendre un ou plusieurs tensioactifs.

Les tensioactifs peuvent être de toute nature mais de préférence cationiques et/ou non ioniques.

La taille moyenne en nombre des particules de silicone dans l'émulsion va généralement de 3 nm à 500 nanomètres, de préférence de 5 nm à 300 nanomètres et plus particulièrement de 10 nm à 275 nanomètres et encore plus préférentiellement de 150 à 275 nanomètres.

Une silicone répondant à cette formule est par exemple la DC2-8566 Amino fluid de DOW CORNING.

c) les silicones aminées répondant à la formule (H) :

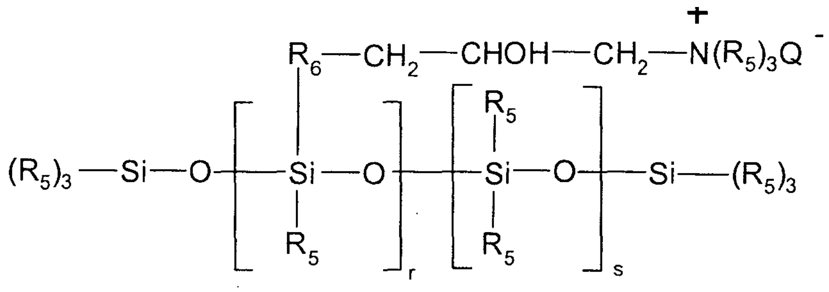

(H)

dans laquelle :

$R_5$ représente un radical hydrocarboné monovalent ayant de 1 à 18 atomes de carbone, et en particulier un radical alkyle en $C_1$-$C_{18}$, ou alcényle en $C_2$-$C_{18}$, par exemple méthyle ;
$R_6$ représente un radical hydrocarboné divalent, notamment un radical alkylène en $C_1$-$C_{18}$ ou un radical alkylèneoxy divalent en $C_1$-$C_{18}$, par exemple en $C_1$-$C_8$ relié au Si par une liaison SiC;
Q- est un anion tel qu'un ion halogénure, notamment chlorure ou un sel d'acide organique (acétate ...);
r représente une valeur statistique moyenne de 2 à 20 et en particulier de 2 à 8 ;
s représente une valeur statistique moyenne de 20 à 200 et en particulier de 20 à 50.

De telles silicones aminées sont décrites plus particulièrement dans le brevet US 4 185 087.
d) les silicones ammonium quaternaire de formule :

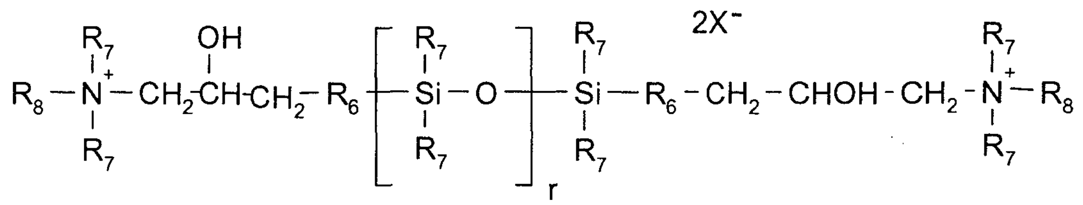

(I)

dans laquelle :

$R_7$, identiques ou différents, représentent un radical hydrocarboné monovalent ayant de 1 à 18 atomes de carbone, et en particulier un radical alkyle en $C_1$-$C_{18}$, un radical alcényle en $C_2$-$C_{18}$ ou un cycle comprenant 5 ou 6 atomes de carbone, par exemple méthyle ;
$R_6$ représente un radical hydrocarboné divalent, notamment un radical alkylène en $C_1$-$C_{18}$ ou un radical alkylèneoxy divalent en $C_1$-$C_{18}$, par exemple en $C_1$-$C_8$ relié au Si par une liaison SiC ;
$R_8$, identiques ou différents, représentent un atome d'hydrogène, un radical hydrocarboné monovalent ayant de 1 à 18 atomes de carbone, et en particulier un radical alkyle en $C_1$-$C_{18}$, un radical alcényle en $C_2$-$C_{18}$, un radical -$R_6$-NHCO$R_7$ ;
X- est un anion tel qu'un ion halogénure, notamment chlorure ou un sel d'acide organique (acétate ...);
r représente une valeur statistique moyenne de 2 à 200 et en particulier de 5 à 100 ;
Ces silicones sont par exemple décrites dans la demande EP-A-0530974.

e) les silicones aminées de formule :

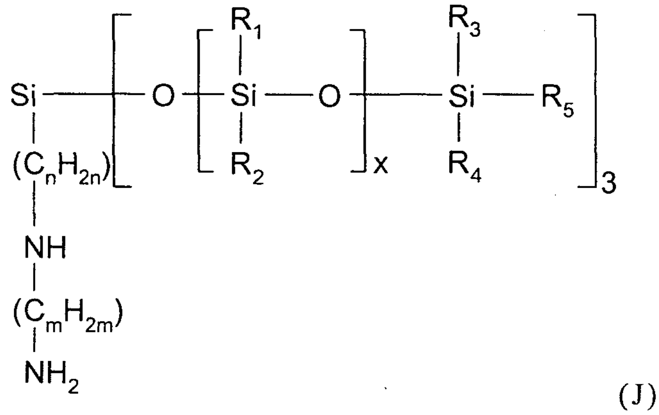

(J)

dans laquelle :

- $R_1$, $R_2$, $R_3$ et $R_4$, identiques ou différents, désignent un radical alkyle en $C_1$-$C_4$ ou un groupement phényle,
- $R_5$ désigne un radical alkyle en $C_1$-$C_4$ ou un groupement hydroxyle,
- n est un entier variant de 1 à 5,
- m est un entier variant de 1 à 5,

et dans laquelle x est choisi de manière telle que l'indice d'amine varie de 0,01 à 1 meq/g.

f) les silicones aminées polyoxyalkylénées de type $(XY)_i$ , X étant un bloc polysiloxane et Y étant un bloc polyoxyalkylèné comportant au moins un groupement amine pouvant, de préférence, être constituées d'unités répétitives de formule générale suivante:

$$[SiMe_2\text{-}O\text{-}(SiMe_2O)_xSiMe_2\text{-}R\text{-}N(H)\text{-}R'\text{-}O\text{-}(C_2H_4O)_a\text{-}(C_3H_6O)_b\text{-}R'\text{-}N(H)\text{-}R\text{-}] \qquad K)$$

dans laquelle :

- a est un nombre entier supérieur ou égal à 1, de préférence allant de 5 à 200 et encore plus particulièrement de 5 à 100 ;
- b est un nombre entier allant de 0 à 200, de préférence allant de 4 à 200 et encore plus particulièrement de 5 à 100 ;
- x est un nombre entier allant de 1 à 10000 et encore plus particulièrement de 10 à 5000 ;
- R, identiques ou différents, représentent un groupe organique divalent qui est lié à l'atome de silicium adjacent par une liaison carbone-silicium et à un atome d'azote,
- R', identiques ou différents, représentent un groupe organique divalent qui est lié à l'atome d'oxygène adjacent par une liaison carbone-oxygène et à un atome d'azote,

R est de préférence un radical hydrocarboné en $C_2$-$C_{12}$ comportant éventuellement un ou plusieurs hétéroatomes tels que l'oxygène. Plus particulièrement, R désigne un radical éthylène, propylène linéaire ou ramifié, butylène linéaire ou ramifié ou $-CH_2CH_2CH_2OCH(OH)CH_2-$.

R' est de préférence un radical hydrocarboné en $C_2$-$C_{12}$ comportant éventuellement un ou plusieurs hétéroatomes tels que l'oxygène. Plus particulièrement R' désigne un radical alkylène divalent comme par exemple l'éthylène, le propylène linéaire ou ramifié ou le butylène linéaire ou ramifié.

Les blocs siloxane représentent généralement de 50 à 95% en moles par rapport au poids total de la silicone et plus particulièrement de 70 à 85% en moles.

Le taux d'amine va généralement de 0,02 à 0,5 meq/g de copolymère dans une solution à 30% dans le dipropylèneglycol et plus particulièrement de 0,05 à 0,2.

Le poids moléculaire moyen en poids de la silicone de formule (K) est de préférence allant de 5000 à 1000000 et encore plus particulièrement de 10000 à 200000.

La silicone particulièrement visée par cette formule (K) est celle commercialisée sous la marque Silsoft A-843 Organosilicone Copolymer par OSI.

Les silicones aminées préférées conformément à l'invention sont celles de formule (A) et (B) et leurs mélanges. Les silicones aminées particulièrement préférées sont celles de formule (A) à (G) et leurs mélanges.

On peut plus particulièrement utiliser les silicones dénommées amodiméthicone et triméthylsilylamodiméthicone selon la dénomination CTFA (2000).

De préférence, l'huile siliconée comprend un polydiméthyl siloxane à groupements aminoéthyl iminoisobutyles. Un tel produit est par exemple commercialisé sous la dénomination DC2-8566 AMINO FLUID par la société DOW CORNING.

La composition selon l'invention comprend de préférence au moins 0,01% en poids d'huile, par rapport au poids total de la composition. De manière préférée, elle comprend de 0,01 à 20% en poids d'huile, plus préférentiellement de 0,1 à 10% en poids, encore plus préférentiellement de 0,2 à 5% en poids, et mieux encore de 0,5 à 3% en poids d'huile, par rapport au poids total de la composition.

La composition objet de la présente invention comprend également du mono-laurate de sorbitan oxyéthylène à 4 OE.

Selon un mode de réalisation préféré, la composition selon l'invention comprend un mélange de mono-laurate de sorbitan oxyéthylène à 4 OE et de mono-laurate de sorbitan oxyéthylène à 20 OE.

La composition selon l'invention comprend avantageusement une quantité totale d'au moins 0,5 % en poids d'ester(s) de sorbitan oxyéthyléné(s) par rapport au poids total de la composition. De préférence, elle comprend une quantité totale d'ester(s) de sorbitan oxyéthyléné(s) allant de 0,5 à 20 % en poids, plus préférentiellement de 2 à 15 % en poids, et encore plus préférentiellement de 4 à 10 % en poids, par rapport au poids total de la composition.

La composition selon l'invention comprend préférentiellement de 2 à 10 % en poids de mono-laurate de sorbitan oxyéthylène à 4 OE.

Lorsque le mono-laurate de sorbitan oxyéthylène à 20 OE est présent, la composition selon l'invention comprend préférentiellement de 1 à 3 % en poids de ce composé.

La composition selon l'invention comprend également au moins un tensioactif anionique.

Les tensioactifs anioniques pouvant être utilisés dans les compositions selon l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les $\alpha$-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en $C_{6-24}$ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en $C_{6-24}$)éther-carboxyliques polyoxyalkylénés, les acides (alkyl en $C_{6-24}$)(aryl en $C_{6-24}$)éther-carboxyliques polyoxyalkylénés, les acides (alkyl en $C_{6-24}$)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et leurs mélanges, en particulier sous forme de sels de métaux alcalins ou alcalino-terreux, d'ammonium, d'amine ou d'aminoalcool.

De préférence, la composition selon l'invention comprend de 0,5 à 50 % en poids de tensioactif(s) anionique(s), plus préférentiellement de 4 à 20% en poids, par rapport au poids total de la composition.

La composition selon l'invention comprend également un ou plusieurs tensioactifs amphotères ou zwittérioniques.

Les agents tensioactifs amphotères ou zwittérioniques utilisables dans les compositions selon l'invention peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer également les alkyl($C_{8-20}$)bétaïnes, les sulfobétaïnes, les (alkyl en $C_{8-20}$)amido(alkyl en $C_{6-8}$)bétaïnes ou les (alkyl en $C_{8-20}$)amido(alkyl en $C_{6-8}$)sulfobétaïnes.

Parmi les dérivés d'amines, on peut citer les produits commercialisés sous la dénomination MIRANOL®, tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (I) et (II) :

$$R_a\text{-CONHCH}_2\text{CH}_2\text{-N}(R_b)(R_c)(\text{CH}_2\text{COO}^-) \qquad \text{(I)}$$

dans laquelle :

$R_a$ représente un groupe alkyle dérivé d'un acide $R_a$-COOH présent dans l'huile de coprah hydrolysée, un groupe heptyle, nonyle ou undécyle,
$R_b$ représente un groupe bêta-hydroxyéthyle, et
$R_c$ représente un groupe carboxyméthyle ;

et

$$R_a'\text{-CONHCH}_2\text{CH}_2\text{-N(B)(B')} \qquad \text{(II)}$$

dans laquelle :

B représente -$CH_2CH_2OX'$,
B' représente -$(CH_2)_z$-Y', avec z = 1 ou 2,
X' représente le groupe -$CH_2CH_2$-COOH ou un atome d'hydrogène,
Y' représente -COOH ou le groupe -$CH_2$-CHOH-$SO_3H$,
$R_a$' représente un groupe alkyle d'un acide $R_a$'-COOH présent dans l'huile de coprah ou dans l'huile de lin hydrolysée, un groupe alkyle, notamment en $C_{17}$ et sa forme iso, un groupe en $C_{17}$ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.
A titre d'exemple, on peut citer le cocoamphodiacétate commercialisé par la société RHODIA sous la dénomination commerciale MIRANOL® C2M concentré.
Parmi les tensioactifs amphotères ou zwittérioniques cités ci-dessus, on utilise de préférence les (alkyl en $C_{8-20}$)-bétaïnes, les (alkyl en $C_{8-20}$)-amido(alkyl en $C_{6-8}$)bétaïnes et leurs mélanges.
La quantité du ou des tensioactifs amphotères ou zwittérioniques est de préférence comprise dans l'intervalle allant de 0,1 à 10 % en poids, mieux encore de 0,5 à 8 % en poids par rapport au poids total de la composition.
De préférence, le rapport pondéral entre la concentration en tensioactif(s) amphotère(s) ou zwittérionique(s) d'une part et la concentration en tensioactif(s) anionique(s) d'autre part est compris entre 0,1 et 0,5.
Le rapport pondéral entre la quantité d'huile d'une part et la quantité totale d'ester(s) de sorbitan oxyéthyléné(s), de tensioactif(s) anionique(s) et de tensiocatif(s) amphotère(s) ou zwittérionique(s) d'autre part est de préférence compris entre 0,01 et 0,2, et plus préférentiellement entre 0,02 et 0,1.
La composition selon l'invention peut également comprendre, en plus du (des) ester(s) de sorbitan oxyéthyléné(s) un ou plusieurs autres tensioactifs non-ioniques additionnels, différents de ces derniers.
Des exemples de tensioactifs non-ioniques additionnels utilisables dans les compositions de la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl($C_{1-20}$) phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.
On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du saccharose, les esters d'acides gras du polyéthylèneglycol, les (alkyl en $C_{6-24}$)polyglycosides, les dérivés de N-(alkyl en $C_{6-24}$)glucamine, les oxydes d'amines tels que les oxydes d'(alkyl en $C_{10-14}$)amines ou les oxydes de N-(acyl en $C_{10-14}$)-aminopropylmorpholine.
Lorsqu'ils sont présents, la quantité du ou des tensioactifs non ioniques additionnels est de préférence comprise dans l'intervalle allant de 0,01 à 10 % en poids, mieux encore de 0,05 à 5% en poids par rapport au poids total de la composition.
La composition selon la présente invention peut comprendre en outre au moins un tensioactif cationique.
A titre d'exemples de tensioactif cationique, on peut notamment citer les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline ; ou les oxydes d'amines à caractère cationique.
Lorsque les tensioactifs cationiques sont présents, leur quantité est de préférence comprise dans l'intervalle allant de 0,01 à 10 % en poids, mieux encore de 0,05 à 5 % en poids, et encore plus préférentiellement de 0,3 à 3 %

en poids par rapport au poids total de la composition cosmétique.

Les compositions selon la présente invention peuvent comprendre en outre au moins un polymère cationique.

Par "polymère cationique", on entend tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont une masse moléculaire moyenne en poids supérieure à $10^5$, de préférence supérieure à $10^6$ et mieux encore comprise entre $10^6$ et $10^8$.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont ceux décrits dans les brevets français nos2 505 348 et 2 542 997. Parmi ces polymères, on peut citer :

(1) les homopolymères ou copolymères dérivés d'esters ou d'amides acryliques ou méthacryliques et comportant au moins un des motifs de formules suivantes:

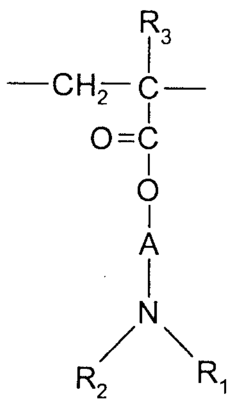
,
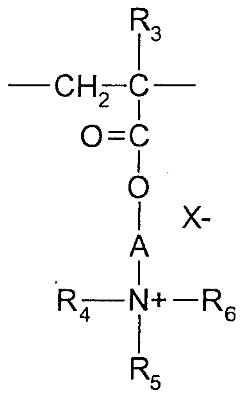

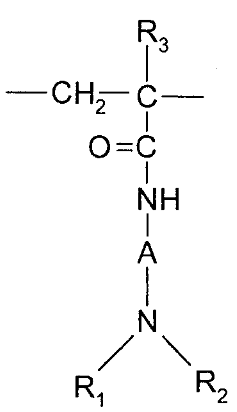
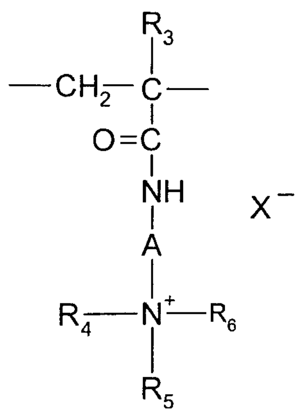

dans lesquelles:

$R_1$ et $R_2$, identiques ou différents, représentent un atome d'hydrogène ou un groupe alkyle ayant de 1 à 6 atomes de carbone, et de préférence un groupe méthyle ou éthyle ;

$R_3$, identiques ou différents, désignent un atome d'hydrogène ou un groupe $CH_3$ ;

les symboles A, identiques ou différents, représentent un groupe alkyle, linéaire ou ramifié, comportant de 1 à 6 atomes de carbone, de préférence 2 ou 3 atomes de carbone, ou un groupe hydroxyalkyle comportant de 1

à 4 atomes de carbone ;

$R_4$, $R_5$, $R_6$, identiques ou différents, représentent un groupe alkyle ayant de 1 à 18 atomes de carbone ou un groupe benzyle, et de préférence un groupe alkyle ayant de 1 à 6 atomes de carbone ;

X désigne un anion dérivé d'un acide minéral ou organique tel qu'un anion méthosulfate ou un halogénure comme le chlorure ou le bromure.

Les copolymères de la famille (1) peuvent contenir en outre un ou plusieurs motifs dérivant de comonomères pouvant être choisis dans la famille des acrylamides, méthacrylamides, diacétone-acrylamides, acrylamides et méthacrylamides substitués sur l'atome d'azote par des groupes alkyle inférieur ($C_1$-$C_4$), des groupes dérivés des acides acryliques ou méthacryliques ou de leurs esters, de vinyllactames tels que la vinylpyrrolidone ou le vinylcaprolactame, d'esters vinyliques.

Ainsi, parmi ces copolymères de la famille (1), on peut citer :

- les copolymères d'acrylamide et de méthacrylate de diméthylaminoéthyle quaternisé au sulfate de diméthyle ou avec un halogénure de diméthyle,
- les copolymères d'acrylamide et de chlorure de méthacryloyl-oxyéthyltriméthylammonium décrits, par exemple, dans la demande de brevet EP-A-080976,
- les copolymères d'acrylamide et de méthosulfate de méthacryloyloxyéthyltriméthylammonium,
- les copolymères vinylpyrrolidone/acrylate ou méthacrylate de dialkylaminoalkyle quaternisés ou non. Ces polymères sont décrits en détail dans les brevets français 2 077 143 et 2 393 573,
- les terpolymères méthacrylate de diméthylaminoéthyle/ vinylcaprolactame/vinylpyrrolidone,
- les copolymères vinylpyrrolidone/méthacrylamidopropyl-diméthylamine, et
- les copolymères vinylpyrrolidone/méthacrylamide de diméthylaminopropyle quaternisé.

(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammoniums quaternaires d'hydroxyéthylcellulose ayant réagi avec un époxyde substitué par un groupement triméthylammonium.

(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium, de diméthyldiallylammonium.

Les produits commercialisés répondant à cette définition sont plus particulièrement les produits vendus sous la dénomination "Celquat® L 200" et "Celquat® H 100" par la Société National Starch.

(4) Les polysaccharides cationiques non cellulosiques décrits dans les brevets US 3 589 578 et 4 031 307 tels que les gommes de guar contenant des groupements cationiques trialkylammonium. On utilise, par exemple, des gommes de guar modifiées par un sel, par exemple le chlorure, de 2,3-époxypropyltriméthylammonium.

De tels produits sont commercialisés notamment sous les dénominations commerciales de JAGUAR® C13 S, JAGUAR® C15, JAGUAR® C17 ou JAGUAR® C162 par la société MEYHALL.

(5) Les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361.

(6) Les polyaminoamides solubles dans l'eau, préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alkylés ou s'ils comportent une ou plusieurs fonctions amines tertiaires, quaternisées. De tels polymères sont notamment décrits dans les brevets français 2 252 840 et 2 368 508.

(7) Les dérivés de polyaminoamides résultant de la condensation de polyalkylènes-polyamines avec des acides polycarboxyliques, suivie d'une alkylation par des agents bifonctionnels. On peut citer, par exemple, les polymères acide adipique/diakylaminohydroxyalkyl-dialkylènetriamine dans lesquels le groupe alkyle comporte de 1 à 4 atomes

de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.

Parmi ces dérivés, on peut citer plus particulièrement les polymères acide adipique/diméthylaminohydroxypropyl-diéthylène-triamine.

(8) Les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347.

(9) Les cyclopolymères d'alkyldiallylamine ou de dialkyldiallylammonium tels que les homopolymères ou copolymères comportant, comme constituant principal de la chaîne, des motifs répondant aux formules (Va) ou (Vb) :

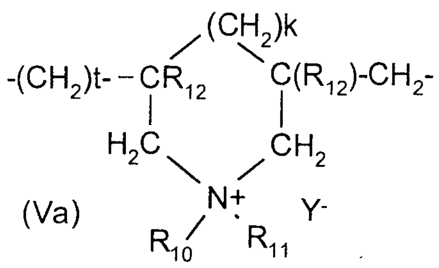
(Va)

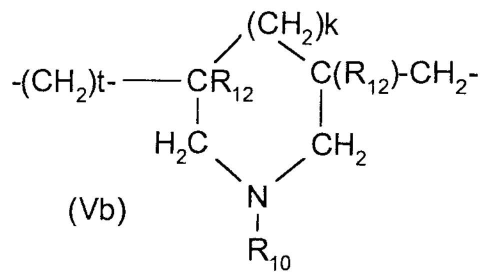
(Vb)

dans lesquelles k et t sont égaux à 0 ou 1, la somme k + t étant égale à 1 ; $R_{12}$ désigne un atome d'hydrogène ou un groupe méthyle ; $R_{10}$ et $R_{11}$, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur ($C_1$-$C_4$), ou alors $R_{10}$ et $R_{11}$ peuvent désigner conjointement avec l'atome d'azote auquel ils sont rattachés, des groupements hétérocycliques, tels que pipéridinyle ou morpholinyle ; $Y^-$ est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.

$R_{10}$ et $R_{11}$, indépendamment l'un de l'autre, désignent de préférence un groupement alkyle ayant de 1 à 4 atomes de carbone.

Parmi les polymères définis ci-dessus, on peut citer plus particulièrement l'homopolymère de chlorure de diméthyldiallylammonium vendu sous la dénomination "MERQUAT® 100" par la société CALGON (et ses homologues de faibles masses moléculaires moyenne en poids) et les copolymères de chlorure de diallyldiméthylammonium et d'acrylamide commercialisés sous la dénomination "MERQUAT® 550".

(10) Les polymères de diammonium quaternaire contenant des motifs récurrents répondant à la formule (VI) :

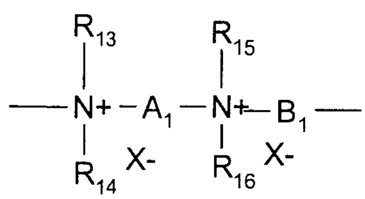
(VI)

dans laquelle :

$R_{13}$, $R_{14}$, $R_{15}$ et $R_{16}$, identiques ou différents, représentent des groupes aliphatiques, alicycliques ou arylaliphatiques contenant de 1 à 20 atomes de carbone ou des groupes hydroxyalkylaliphatiques inférieurs, ou bien $R_{13}$, $R_{14}$, $R_{15}$ et $R_{16}$, ensemble ou séparément, constituent avec les atomes d'azote auxquels ils sont rattachés des hétérocycles contenant éventuellement un second hétéroatome autre que l'azote, ou bien $R_{13}$, $R_{14}$, $R_{15}$ et $R_{16}$ représentent un groupe alkyle en $C_1$-$C_6$, linéaire ou ramifié, substitué par un groupement nitrile, ester, acyle, amide ou -CO-O-$R_{17}$-E ou -CO-NH-$R_{17}$-E où $R_{17}$ est un groupe alkylène et E un groupement ammonium

quaternaire ;

$A_1$ et $B_1$ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et $X^-$ désigne un anion dérivé d'un acide minéral ou organique;

$A_1$, $R_{13}$ et $R_{15}$ peuvent former avec les deux atomes d'azote auxquels ils sont rattachés un cycle pipérazinique ; en outre, si $A_1$ désigne un groupe alkylène ou hydroxyalkylène linéaire ou ramifié, saturé ou insaturé, $B_1$ peut également désigner un groupement :

$$-(CH_2)_n-CO-E'-OC-(CH_2)_n-$$

dans lequel E' désigne :

a) un reste de glycol de formule -O-Z-O-, où Z désigne un groupe hydrocarboné linéaire ou ramifié, ou un groupement répondant à l'une des formules suivantes :

$$-(CH_2-CH_2-O)_x-CH_2-CH_2-$$

$$-[CH_2-CH(CH_3)-O]_y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;

b) un reste de diamine bis-secondaire tel qu'un dérivé de pipérazine ;

c) un reste de diamine bis-primaire de formule -NH-Y-NH-, où Y désigne un groupe hydrocarboné linéaire ou ramifié, ou bien le groupe divalent $-CH_2-CH_2-S-S-CH_2-CH_2-$ ;

d) un groupement uréylène de formule -NH-CO-NH-.

De préférence, $X^-$ est un anion tel que le chlorure ou le bromure.

Des polymères de ce type sont notamment décrits dans les brevets français 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.On peut utiliser plus particulièrement les polymères qui sont constitués de motifs récurrents répondant à la formule :

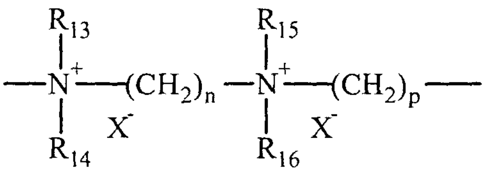 (VII)

dans laquelle $R_{13}$, $R_{14}$, $R_{15}$ et $R_{16}$, identiques ou différents, désignent un groupe alkyle ou hydroxyalkyle ayant de 1 à 4 atomes de carbone environ, n et p sont des nombres entiers variant de 2 à 20 environ et, $X^-$ est un anion dérivé d'un acide minéral ou organique.

(11) Les polymères de polyammonium quaternaire constitués de motifs de formule (VIII) :

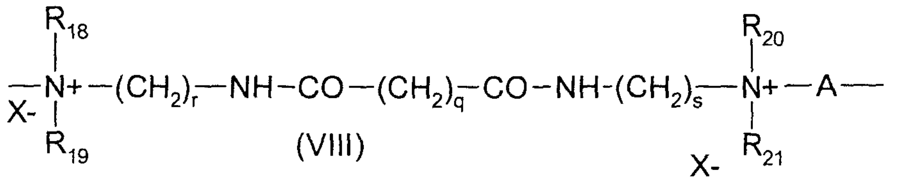

(VIII)

dans laquelle :

$R_{18}$, $R_{19}$, $R_{20}$ et $R_{21}$, identiques ou différents, représentent un atome d'hydrogène ou un groupe méthyle, éthyle, propyle, β-hydroxyéthyle, β-hydroxypropyle ou $-CH_2CH_2(OCH_2CH_2)_pOH$, où p est égal à 0 ou à un nombre entier compris entre 1 et 6, sous réserve que $R_{18}$, $R_{19}$, $R_{20}$ et $R_{21}$ ne représentent pas simultanément un atome d'hydrogène,
r et s, identiques ou différents, sont des nombres entiers compris entre 1 et 6,
q est égal à 0 ou à un nombre entier compris entre 1 et 34,
$X^-$ désigne un anion tel qu'un halogénure,
A désigne un radical d'un dihalogénure ou représente de préférence $-CH_2-CH_2-O-CH_2-CH_2-$.

De tels composés sont notamment décrits dans la demande de brevet EP-A-122 324.
(12) Les polymères quaternaires de vinylpyrrolidone et de vinylimidazole.
(13) Les polymères réticulés de sels de méthacryloyloxyalkyl($C_1$-$C_4$) trialkyl($C_1$-$C_4$)ammonium tels que les polymères obtenus par homopolymérisation du méthacrylate de diméthylaminoéthyle quaternisé par le chlorure de méthyle, ou par copolymérisation de l'acrylamide avec le méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bisacrylamide.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose comportant des groupements ammonium quaternaires tels que les produits vendus sous la dénomination "JR 400" par la Société UNION CARBIDE CORPORATION, les cyclopolymères cationiques, en particulier les homopolymères ou copolymères de chlorure de diméthyldiallylammonium, vendus sous les dénominations MERQUAT® 100, MERQUAT® 550 et MERQUAT® S par la société CALGON, les gommes de guar modifiées par un sel de 2,3-époxypropyl-triméthylammonium, les polymères quaternaires de vinylpyrrolidone et de vinylimidazole.

Lorsque le(s) polymère(s) cationique(s) est (sont) présent(s), la composition selon l'invention comprend de 0,01 à 10 % en poids de polymère(s) cationique(s), mieux encore de 0,1 à 5 % en poids, et encore plus préférentiellement de 0,1 à 2 % en poids, par rapport au poids total de la composition.

La composition selon l'invention peut comprendre en outre au moins un agent antipelliculaire. Comme agents antipelliculaires, peuvent être employés par exemple des composés tels que la piroctone olamine, la pyrithione de zinc, l'acide salicylique, le disulfure de sélénium, et leurs mélanges.

La composition comprend alors de 0,001 à 10 % en poids d'agent(s) antipelliculaire(s), de préférence de 0,1 à 5 % en poids, encore plus préférentiellement de 0,2 à 2 % en poids, par rapport au poids total de la composition.

Le pH de la composition selon l'invention est généralement inférieur à 8,5, et de préférence compris dans l'intervalle allant de 4 à 7.

La composition selon l'invention peut comprendre en outre un ou plusieurs additifs classiques bien connus dans la technique, tels que les agents antichute, les agents oxydants, les céramides et pseudo-céramides, les vitamines et pro-vitamines dont le panthénol, les huiles végétales, animales, minérales ou synthétiques, les cires, les céramides et pseudo-céramides, les filtres solaires, les pigments minéraux ou organiques, colorés ou non colorés, les colorants, les agents séquestrants, les agents plastifiants, les agents solubilisants, les agents acidifiants, des agents alcalinisants, les agents épaississants minéraux ou organiques, les agents anti-oxydants, les hydroxyacides, les parfums et les agents conservateurs.

Comme agents épaississants, peuvent être employés en particulier des dérivés de la cellulose, tels que par exemple la carboxyméthylcellulose, l'hydroxypropyl-cellulose, l'hydroxyéthylcellulose, la gomme de guar, les gommes de guar hydroxypropylées, les scléroglucanes, la gomme de xanthane, les polymères amphiphiles comportant au moins une chaîne grasse, les amides gras tels que la monoéthanolamide d'acide de coco, la monoisopropanolamide d'acide de coco.

L'homme de métier veillera à choisir les éventuels additifs et leurs quantités de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

Les compositions selon l'invention peuvent se présenter sous la forme de gels douches, de shampooings, de compositions à appliquer avant ou après un shampooing.

La composition selon l'invention peut être utilisée notamment comme composition de traitement ou de soin

cosmétique des matières kératiniques, telles que par exemple les cheveux ou la peau. En particulier, elle peut être utilisée comme gel douche ou comme shampooing, et de préférence comme shampooing.

Un autre objet de l'invention est un procédé de traitement cosmétique des matières kératiniques, de préférence les cheveux, par exemple un procédé de lavage des cheveux. Ce procédé comprend l'application sur les matières kératiniques d'une quantité efficace d'une composition cosmétique telle que décrite ci-dessus.

Selon un mode de mise en œuvre préféré, un tel procédé consiste à appliquer sur les matières kératiniques une quantité efficace de la composition cosmétique, à éventuellement rincer après un éventuel temps de pause.

Les exemples suivants sont donnés à titre illustratif de la présente invention, et ne sauraient en limiter la portée.

EXEMPLES

Dans les exemples ci-après, toutes les quantités sont indiquées en pour cent en poids de matière active par rapport au poids total de la composition, sauf indication contraire.

Exemple 1 :

Une composition de shampooing conforme à l'invention, a été préparée à partir des ingrédients indiqués dans le tableau ci-dessous.

| Composition | |
|---|---|
| amodiméthicone (DC 2-8566 amino fluid de Dow Corning) | 1 |
| hexylène glycol | 0,8 |
| acide carboxylique de PEG-5 lauryl éther (Akypo RLM 45 CA de Kao) | 0,9 |
| lauryl éther sulfate de sodium à 2,2 OE en solution aqueuse (Texapon N702 de Cognis) | 11,2 |
| lauryl bétaïne (Empigen BB/LS de Huntsman) | 5,1 |
| polyquaternium-10 (Polyquta 400 KC de KCI) | 0,4 |
| chlorure de sodium | 0,2 |
| mono-laurate de sorbitan oxyéthylène à 4 OE (Tween 21 de Uniqema) | 4 |
| monolaurate de sorbitan oxyéthylène à 20 OE (Tween 20 de Uniqema) | 1,3 |
| Agents conservateurs, parfum | 0,5 |
| Eau | qsp 100 |

Cette composition a été préparée de la manière suivante :

Le polyquaternium-10 est dispersé dans de l'eau, sous agitation, puis le lauryl éther sulfate de sodium est ajouté, et solubilisé sous agitation.
On ajoute alors l'acide carboxylique de PEG-5 lauryl éther, prémélangé aux agents conservateurs et au parfum, et chauffé à 70°C.
Sont ensuite successivement ajoutés dans le mélange, et solubilisés sous agitation : l'amodiméthicone, le monolaurate de sorbitan oxyéthylène à 20 OE prémélangé à l'hexylène glycol, le mono-laurate de sorbitan oxyéthylène à 4 OE, et enfin la lauryl bétaïne.
On complète le mélange à l'eau (qsp 100% en poids).

Exemple 2 :

Une composition de shampooing conforme à l'invention, a été préparée à partir des ingrédients indiqués dans le tableau ci-dessous.

| Composition | |
|---|---|
| amodiméthicone (DC 2-8566 amino fluid de Dow Corning) | 1 |
| hexylène glycol | 1,6 |

(suite)

| Composition | |
|---|---|
| acide carboxylique de PEG-5 lauryl éther (Akypo RLM 45 CA de Kao) | 0,9 |
| lauryl éther sulfate de sodium à 2,2 OE en solution aqueuse (Texapon N702 de Cognis) | 11,2 |
| lauryl bétaïne (Empigen BB/LS de Huntsman) | 5,1 |
| polyquaternium-10 (Polyquta 400 KC de KCI) | 0,4 |
| chlorure de sodium | 0,2 |
| mono-laurate de sorbitan oxyéthylène à 4 OE (Tween 21 de Uniqema) | 8 |
| monolaurate de sorbitan oxyéthylène à 20 OE (Tween 20 de Uniqema) | 2,7 |
| Agents conservateurs, parfum | 0,5 |
| Eau | qsp 100 |

Cette composition est préparée de manière identique à la composition de l'exemple 1.

Exemple 3 :

Une composition de shampooing conforme à l'invention, a été préparée à partir des ingrédients indiqués dans le tableau ci-dessous.

| Composition | |
|---|---|
| lauryl éther sulfate de sodium à 2,2 OE en solution aqueuse (Texapon N702 de Cognis) | 11,2 |
| lauryl bétaïne (Empigen BB/LS de Huntsman) | 5,1 |
| mono-laurate de sorbitan oxyéthylène à 4 OE (Tween 21 de Uniqema) | 6 |
| monolaurate de sorbitan oxyéthylène à 20 OE (Tween 20 de Uniqema) | 2 |
| amodiméthicone (DC 2-8566 amino fluid de Dow Corning) | 1 |
| acide carboxylique de PEG-5 lauryl éther (Akypo RLM 45 CA de Kao) | 0,9 |
| parfum | 0,5 |
| mélange de p-hydroxybenzoates de méthyle, butyle, éthyle, propyle, isobutyle (Nipastat de Nipa) | 0,5 |
| hexylène glycol | 1,2 |
| polyquaternium-10 (Polyquta 400 KC de KCI) | 0,4 |
| acide salicylique en poudre | 0,2 |
| benzoate de sodium | 0,5 |
| chlorure de sodium | 0,2 |
| Eau | qsp 100 |

Cette composition est préparée de manière analogue à la composition de l'exemple 1.

Exemple 4 :

Une composition de shampooing conforme à l'invention, a été préparée à partir des ingrédients indiqués dans le tableau ci-dessous.

| Composition | |
|---|---|
| amodiméthicone (DC 2-8566 amino fluid de Dow Corning) | 1 |

(suite)

| Composition | |
|---|---|
| hexylène glycol | 1,2 |
| acide carboxylique de PEG-5 lauryl éther (Akypo RLM 45 CA de Kao) | 0,9 |
| lauryl éther sulfate de sodium à 2,2 OE en solution aqueuse (Texapon N702 de Cognis) | 11,2 |
| lauryl bétaïne (Empigen BB/LS de Huntsman) | 5,1 |
| polyquaternium-10 (Polyquta 400 KC de KCI) | 0,4 |
| oléate de propylène glycol PEG-55 (Antil 141 liquid de Goldschmidt) | 0,3 |
| mono-laurate de sorbitan oxyéthylène à 4 OE (Tween 21 de Uniqema) | 6 |
| Agents conservateurs, parfum | 0,5 |
| Eau | qsp 100 |

Cette composition est préparée de manière analogue à la composition de l'exemple 1.

Les compositions préparées selon les exemples 1 à 4 ci-avant se sont avérées présenter une excellente tolérance vis-à-vis du cuir chevelu.

En outre, ces compositions sont extrêmement douces et présentent une excellente tolérance oculaire.

Enfin, ces compositions présentent de remarquables propriétés cosmétiques de douceur, de démêlage, de lissage des cheveux, de coiffant, de volume et de légèreté.

## Revendications

1. Composition cosmétique pour le lavage des matières kératiniques constituée d'une microémulsion comprenant :

   - de l'eau,
   - du mono-laurate de sorbitan oxyéthylène à 4 OE,
   - au moins une huile choisie parmi les silicones aminées,
   - au moins un tensioactif anionique, et
   - au moins un tensioactif amphotère ou zwittérionique.

2. Composition selon la revendication précédente, **caractérisée en ce qu'**elle comprend au moins une silicone aminée, choisie parmi :

   a) les polysiloxanes répondant à la formule (A):

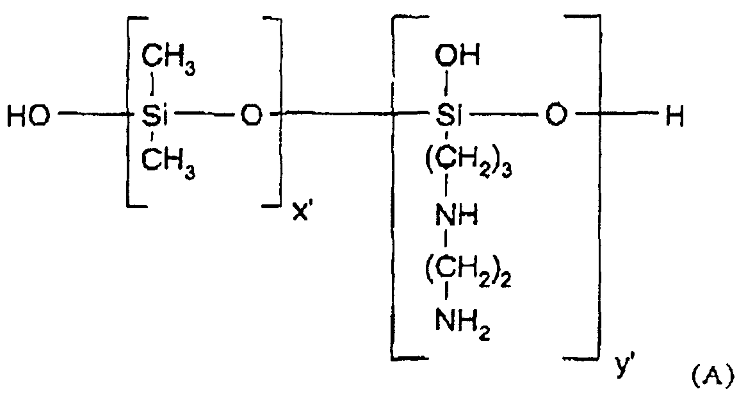

(A)

dans laquelle x' et y' sont des nombres entiers dépendant du poids moléculaire, généralement tels que ledit poids moléculaire moyen en poids est allant de 5 000 et 500 000 ;

b) les silicones aminées répondant à la formule :

$$R'_aG_{3-a}\text{-}Si(OSiG_2)_n\text{-}(OSiG_bR'_{2-b})_m\text{-}O\text{-}SiG_{3-a}\text{-}R'_a \qquad (B)$$

dans laquelle :

G, identiques ou différents, désignent un atome d'hydrogène, ou un groupement phényle, OH, alkyl en $C_1$-$C_8$, ou alcoxy en $C_1$-$C_8$,
a identiques ou différents, désignent le nombre 0 ou un nombre entier de 1 à 3,
b désigne 0 ou 1,
m et n sont des nombres tels que la somme (n+m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R', identiques ou différents, désignent un radical monovalent de formule -CqH2qL dans laquelle q est un nombre de 2 à 8 et L est un groupement aminé éventuellement quaternisé choisi parmi les groupements :

- $NR''\text{-}Q\text{-}N(R'')_2$
- $N(R'')_2$
- $N^-(R'')_3$ A-
- $N^+H(R'')_2$ A-
- $N^+H_2(R'')$ A-
- $N(R'')\text{-}Q\text{-}N^+R''H_2$ A-
- $NR''\text{-}Q\text{-}N^+(R'')_2H$ A-
- $NR''\text{-}Q\text{-}N^+(R'')_3$ A-,

dans lesquels R" peut désigner hydrogène, phényle, benzyle, ou un radical hydrocarboné saturé monovalent, Q désigne un groupement de formule $C_rH_{2r}$, linéaire ou ramifié, r étant un entier allant de 2 à 6, de préférence de 2 à 4 ; et A- représente un ion halogénure ;

c) les silicones aminées répondant à la formule (H) :

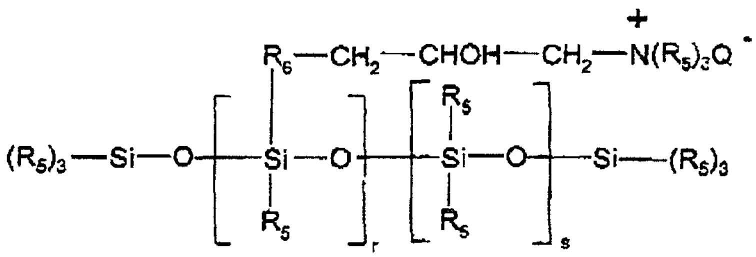

(H)

dans laquelle:

$R_5$ représente un radical hydrocarboné monovalent ayant de 1 à 18 atomes de carbone,
$R_6$ représente un radical hydrocarboné divalent relié au Si par une liaison SiC,
Q- est un anion tel qu'un ion halogénure ou un sel d'acidc organique,
r représente une valeur statistique moyenne de 2 à 20,
s représente une valeur statistique moyenne de 20 à 200 ;

d) les silicones ammonium quaternaire de formule (I) :

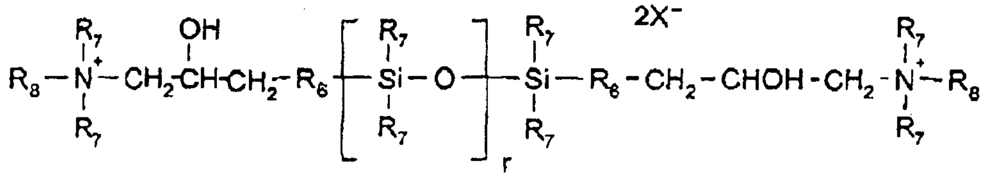

(I)

dans laquelle:

$R_7$, identiques ou différents, représentent un radical hydrocarboné monovalent ayant de 1 à 18 atomes de carbone;
$R_6$ représente un radical hydrocarboné divalent relié au Si par une liaison SiC ;
$R_8$, identiques ou différents, représentent un atome d'hydrogène, un radical hydrocarboné monovalent ayant de 1 à 18 atomes de carbone,
X- est un anion tel qu'un ion halogénure ou un sel d'acide organique,
r représente une valeur statistique moyenne de 2 à 200;

e) les silicones aminées de formule (J):

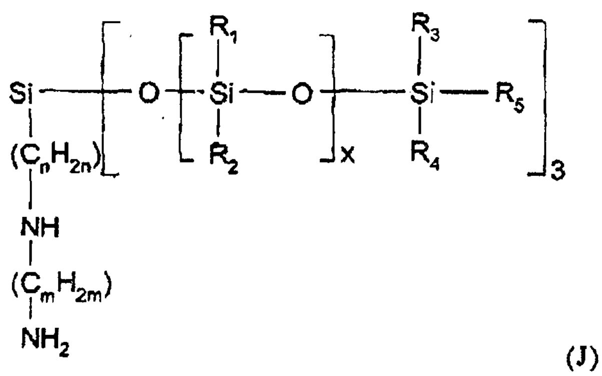

(J)

dans laquelle:

$R_1$, $R_2$, $R_3$ et $R_4$, identiques ou différents, désignent un radical alkyle, en $C_1$-$C_4$ ou un groupement phényle,
$R_5$ désigne un radical alkyle en $C_1$-$C_4$ ou un groupement hydroxyle,
n est un entier variant de 1 à 5,
m est un entier variant de 1 à 5,
et dans laquelle x est choisi de manière telle que l'indice d'amine varie de 0,01 à 1 meq/g ;

f) les silicones aminées polyoxyalkylénées de type $(XY)_i$, X étant un bloc polysiloxane et Y étant un bloc polyoxyalkyléné comportant au moins un groupement amine pouvant, de préférence, être constituées d'unités répétitives de formule générale suivante:

$$[SiMe_2\text{-}O\text{-}(SiMe_2O)_xSiMe_2\text{-}R\text{-}N(H)\text{-}R'\text{-}O\text{-}(C_2H_4O)_a\text{-}(C_3H_6O)_b\text{-}R'\text{-}N(H)\text{-}R\text{-}] \quad (K)$$

dans laquelle :

a est un nombre entier supérieur ou égal à 1, de préférence allant de 5 à 200 et encore plus particulièrement de 5 à 100,
b est un nombre entier allant de 0 à 200, de préférence allant de 4 à 200 et encore plus particulièrement de 5 à 100,
x est un nombre entier allant de 1 à 10000 et encore plus particulièrement de 10 à 5000 ;
R, identiques ou différents, représentent un groupe organique divalent qui est lié à l'atome de silicium adjacent par une liaison carbone-silicium et à un atome d'azote, R', identiques ou différents, représentent un groupe organique divalent qui est lié à l'atome d'oxygène adjacent par une liaison carbone-oxygène et

à un atome d'azote.

**3.** Composition selon la revendication précédente, **caractérisée en ce qu'**elle comprend au moins une silicone aminée choisie parmi celles de formule (A) et (B) et leurs mélanges.

**4.** Composition selon l'une quelconque des revendications 2 et 3, **caractérisée en ce que** l'huile siliconée comprend un polydiméthyl siloxane à groupements aminoéthyl iminoisobutyles.

**5.** Composition selon l'une quelconque des revendications précédentes, **caractérisée en ce qu'**elle comprend au moins 0,01% en poids d'huile, par rapport au poids total de la composition.

**6.** Composition selon la revendication précédente, **caractérisée en ce qu'**elle comprend de 0,01 à 20% en poids d'huile, et de préférence de 0,1 à 10% en poids d'huile, par rapport au poids total de la composition.

**7.** Composition selon l'une quelconque des revendications précédentes, **caractérisée en ce qu'**elle comprend un mélange de mono-laurate de sorbitan oxyéthylène à 4 OE et de mono-laurate de sorbitan oxyéthylène à 20 OE.

**8.** Composition selon l'une quelconque des revendications précédentes, **caractérisée en ce qu'**elle comprend une quantité totale d'au moins 0,5 % en poids d'ester(s) de sorbitan oxyéthyléné(s) par rapport au poids total de la composition.

**9.** Composition selon la revendication précédente, **caractérisée en ce qu'**elle comprend une quantité totale d'ester(s) de sorbitan oxyéthyléné(s) allant de 0,5 à 20 % en poids, plus préférentiellement de 2 à 15 % en poids, et encore plus préférentiellement de 4 à 10 % en poids, par rapport au poids total de la composition.

**10.** Composition selon l'une quelconque des revendications précédentes, **caractérisée en ce qu'**elle comprend au moins un tensioactif anionique choisi parmi les sels des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les $\alpha$-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

**11.** Composition selon l'une quelconque des revendications précédentes, **caractérisée en ce qu'**elle comprend au moins un tensioactif anionique choisi parmi les monoesters d'alkyle en $C_{6-24}$ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

**12.** Composition selon l'une quelconque des revendications précédentes, **caractérisée en ce qu'**elle comprend au moins un tensioactif anionique choisi parmi les acyl-lactylates dont le groupe acyle comporte de 8 à 20 atomes de carbone, les acides alkyl-D-galactoside-uroniques, les acides (alkyl en $C_{6-24}$)éther-carboxyliques polyoxyalkylénés, les acides (alkyl en $C_{6-24}$)(aryl en $C_{6-24}$)éther-carboxyliques polyoxyalkylénés, les acides (alkyl en $C_{6-24}$)amidoéther-carboxyliques polyoxyalkylénés et les sels de ces composés.

**13.** Composition selon la revendication 10, **caractérisée en ce qu'**elle comprend au moins un tensioactif anionique choisi panni les alkylsulfates, les alkyléthersulfates et leurs mélanges, en particulier sous forme de sels de métaux alcalins ou alcalino-terreux, d'ammonium, d'amine ou d'aminoalcool.

**14.** Composition selon l'une quelconque des revendications précédentes, **caractérisée** en cc qu'elle comprend de 0,5 à 50 % en poids de tensioactif(s) anionique(s), de préférence de 4 à 20% en poids, par rapport au poids total de la composition.

**15.** Composition selon l'une quelconque des revendications précédentes, **caractérisée en ce qu'**elle comprend au moins un tensioactif amphotère ou zwittérionique choisi parmi :

- les dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel

que, par exemple, un groupe carboxylate, sulfonate, sulfate, phosphate ou phosphonate, et
- les alkyl($C_{8-20}$)bétaïnes, les sulfobétaïnes, les (alkyl en $C_{8-20}$)amido(alkyl en $C_{6-8}$)bétaïnes ou les (alkyl en $C_{8-20}$)amido(alkyl en $C_{6-8}$)sulfobétaines.

**16.** Composition selon l'une quelconque des revendications précédentes, **caractérisée en ce qu'**elle comprend de 0,1 à 10 % en poids de tensioactif(s) amphotère(s) ou zwittérionique(s), de préférence de 0,5 à 8 % en poids, par rapport au poids total de la composition.

**17.** Composition selon l'une quelconque des revendications 1 et/ou 2 à 14, **caractérisée en ce que** le rapport pondéral entre la quantité d'huile d'une part et la quantité totale d'ester(s) de sorbitan oxyéthyléné(s), de tensioactif(s) anionique(s) et de tensiocatif(s) amphotère(s) ou zwittérionique(s) d'autre part est compris entre 0,01 et 0,2 et plus préférentiellement entre 0,02 et 0,1.

**18.** Composition selon l'une quelconque des revendications précédentes, **caractérisée en ce que** le rapport pondéral entre la concentration en tensioactif(s) amphotère(s) ou zwittérionique(s) d'une part et la concentration en tensioactif(s) anionique(s) d'autre part est compris entre 0,1 et 0,5.

**19.** Composition selon l'une quelconque des revendications précédentes, **caractérisée en ce qu'**elle comprend au moins un tensioactif cationique.

**20.** Composition selon la revendication précédente, **caractérisée en ce que** la quantité de tensioactif(s) cationique(s) est comprise dans l'intervalle allant de 0,01 à 10% en poids, de préférence de 0,05 à 5% en poids, par rapport au poids total de la composition.

**21.** Composition selon l'une quelconque des revendications précédentes, **caractérisée en ce qu'**elle comprend au moins un polymère cationique.

**22.** Composition selon la revendication précédente, **caractérisée en ce qu'**elle comprend de 0,01 à 10% en poids de polymère(s) cationique(s), par rapport au poids total de la composition.

**23.** Composition selon l'une quelconque des revendications précédentes, **caractérisée en ce qu'**elle comprend en outre au moins un agent antipelliculaire, tel que par exemple la piroctone olamine, la pyrithione de zinc, l'acide salicylique, le disulfure de sélénium, et leurs mélanges.

**24.** Composition selon la revendication précédente, **caractérisée en ce qu'**elle comprend de 0,001 à 10 % en poids d'agent(s) antipelliculaire(s), de préférence de 0,1 à 5 % en poids par rapport au poids total de la composition.

**25.** Composition selon l'une quelconque des revendications précédentes, **caractérisée en ce qu'**elle comprend en outre au moins un additif choisi parmi les agents antichute, les agents oxydants, les céramides et pseudo-céramides, les vitamines et pro-vitamines dont le panthénol, les huiles végétales, animales, minérales ou synthétiques, les cires, les céramides et pseudo-céramides, les filtres solaires, les pigments minéraux ou organiques, colorés ou non colorés, les colorants, les agents séquestrants, les agents plastifiants, les agents solubilisants, les agents acidifiants, des agents alcalinisants, les agents épaississants minéraux ou organiques, les agents anti-oxydants, les hydroxyacides, les parfums et les agents conservateurs.

**26.** Utilisation de la composition selon l'une quelconque des revendications 1 à 25, comme composition de traitement ou de soin cosmétique des cheveux ou de la peau.

**27.** Utilisation de la composition selon l'une quelconque des revendications 1 à 25 comme shampooing.

**28.** Procédé de traitement cosmétique des matières kératiniques, **caractérisé en ce qu'**il comprend l'application sur lesdites matières d'une quantité efficace d'une composition selon l'une quelconque des revendications 1 à 25.

## Patentansprüche

**1.** Kosmetische Zusammensetzung zum Waschen von Keratinmaterialien, bestehend aus einer Mikroemulsion, umfassend:

- Wasser,
- mit 4 EO oxyethyleniertes Sorbitanmonolaurat,
- mindestens ein Öl, das aus Aminosilikonen ausgewählt ist,
- mindestens ein anionisches Tensid und
- mindestens ein amphoteres oder zwitterionisches Tensid.

**2.** Zusammensetzung nach dem vorhergehenden Anspruch, **dadurch gekennzeichnet, dass** sie mindestens ein Aminosilikon umfasst, das ausgewählt ist aus:

a) Polysiloxanen der Formel (A):

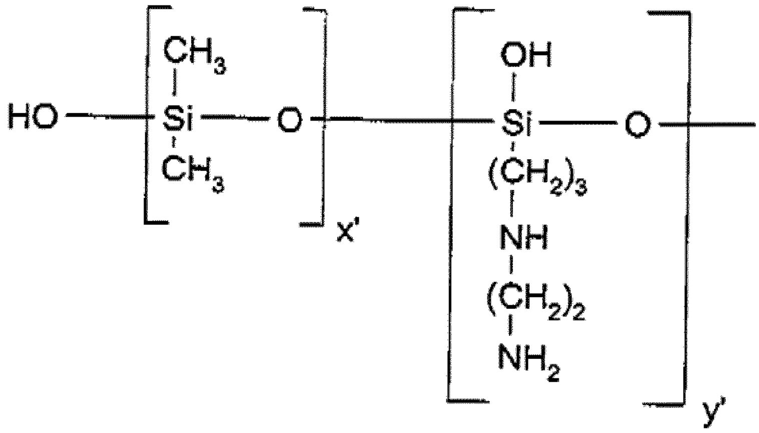
(A)

worin x' und y' vom Molekulargewicht abhängende ganze Zahlen sind, im Allgemeinen derart, dass das gewichtsmittlere Molekulargewicht im Bereich von 5000 bis 500.000 liegt;
b) Aminosilikonen der Formel:

$$R'_aG_{3-a}\text{-Si(OSiG}_2)_n\text{-(OSiG}_bR'_{2-b})_m\text{-O-SiG}_{3-a}\text{-R'}_a \qquad (B)$$

worin:

die Variablen G gleich oder verschieden sind und für ein Wasserstoffatom oder eine Phenyl-, OH-, Ci-Cs-Alkyl- oder
$C_1$-$C_8$-Alkoxygruppe stehen,
die Variablen a gleich oder verschieden sind und für die Zahl 0 oder eine ganze Zahl von 1 bis 3 stehen,
b für 0 oder 1 steht,
m und n solche Zahlen sind, dass die Summe (n + m) insbesondere von 1 bis 2000 und speziell von 50 bis 150 variieren kann, wobei n für eine Zahl von 0 bis 1999 und
insbesondere von 49 bis 149 stehen kann und m für eine Zahl von 1 bis 2000 und insbesondere von 1 bis 10 stehen kann;
die Gruppen R' gleich oder verschieden sind und für einen einwertigen Rest der Formel -CqH2qL stehen, worin q eine Zahl von 2 bis 8 ist und L für eine gegebenenfalls quaternisierte Aminogruppe steht, die ausgewählt ist aus den Gruppen:

- $NR''\text{-}Q\text{-}N(R'')_2$
- $N(R'')_2$
- $N^+(R'')_3$ A-
- $N^+H(R'')_2$ A-
- $N^+H_2(R'')$ A-
- $N(R'')\text{-}Q\text{-}N^+R''H_2$ A-
- $NR''\text{-}Q\text{-}N^+(R'')_2H$ A-
- $NR''\text{-}Q\text{-}N^+(R'')_3$ A-,

worin R" für Wasserstoff, Phenyl, Benzyl oder einen einwertigen gesättigten Kohlenwasserstoffrest stehen

kann, Q für eine lineare oder verzweigte Gruppe der Formel $C_rH_{2r}$ steht, wobei r für eine ganze Zahl im Bereich von 2 bis 6, vorzugsweise von 2 bis 4, steht; und A- für ein Halogenidion steht;

c) Aminosilikonen der Formel (H):

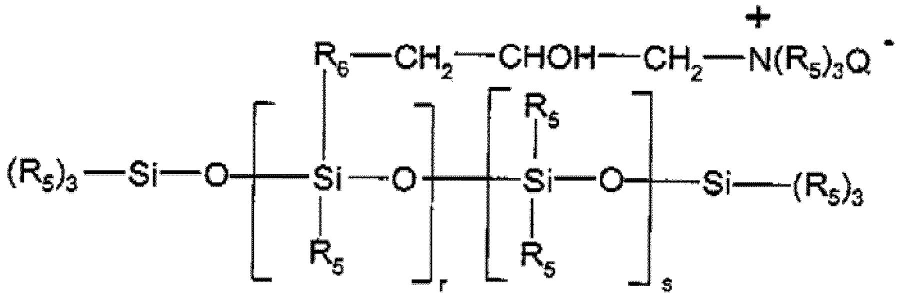

(H)

worin:

$R_5$ für einen einwertigen Kohlenwasserstoffrest mit 1 bis 18 Kohlenstoffatomen steht,
$R_6$ für einen zweiwertigen Kohlenwasserstoffrest steht,
der über eine SiC-Bindung an das Si gebunden ist,
Q- für ein Anion wie ein Halogenidion oder ein Salz einer organischen Säure steht,
r für einen durchschnittlichen statistischen Wert von 2 bis 20 steht,
s für einen durchschnittlichen statistischen Wert von 20 bis 200 steht;

d) quaternären Ammoniumsilikonen der Formel (I):

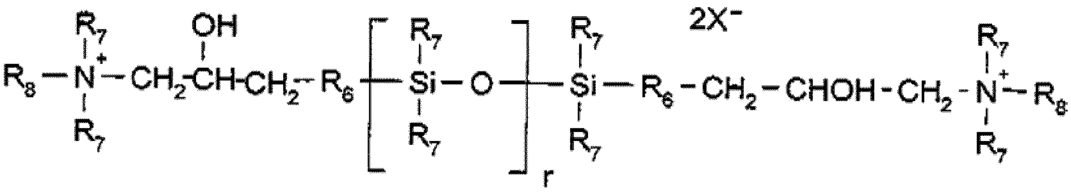

(I)

worin:

die Gruppen $R_7$ gleich oder verschieden sind und für einen einwertigen Kohlenwasserstoffrest mit 1 bis 18 Kohlenstoffatomen stehen;
$R_6$ für einen zweiwertigen Kohlenwasserstoffrest steht,
der über eine SiC-Bindung an das Si gebunden ist;
die Gruppen $R_8$ gleich oder verschieden sind und für ein Wasserstoffatom oder einen einwertigen Kohlenwasserstoffrest mit 1 bis 18 Kohlenstoffatomen stehen,
X- für ein Anion wie ein Halogenidion oder ein Salz einer organischen Säure steht,
r für einen durchschnittlichen statistischen Wert von 2 bis 200 steht;

e) Aminosilikonen der Formel (J):

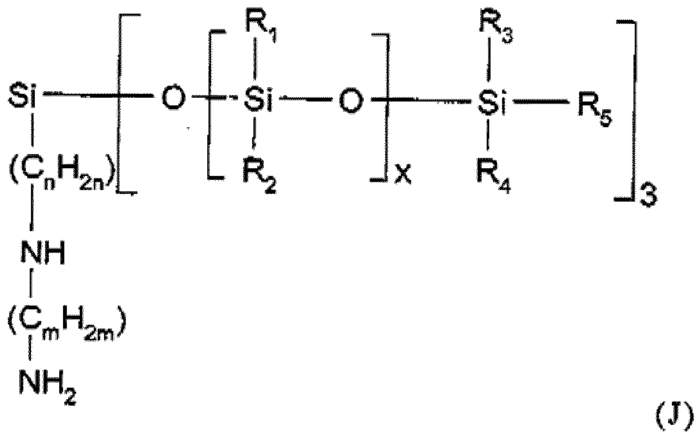

(J)

worin:

$R_1$, $R_2$, $R_3$ und $R_4$ gleich oder verschieden sind und für einen $C_1$-$C_4$-Alkylrest oder eine Phenylgruppe stehen,
$R_5$ für einen $C_1$-$C_4$-Alkylrest oder eine Hydroxylgruppe steht,
n für eine ganze Zahl im Bereich von 1 bis 5 steht,
m für eine ganze Zahl im Bereich von 1 bis 5 steht,
und worin x so gewählt ist, dass die Aminzahl im Bereich von 0,01 bis 1 meq/g liegt;

f) polyoxyalkylenierten Aminosilikonen des Typs $(XY)_i$, wobei X ein Polysiloxanblock ist und Y ein Polyoxyalkylenblock mit mindestens einer Amingruppe ist, die vorzugsweise aus Wiederholungseinheiten der folgenden allgemeinen Formel bestehen können:

$$[SiMe_2\text{-}O\text{-}(SiMe_2O)_xSiMe_2\text{-}R\text{-}N(H)\text{-}R'\text{-}O\text{-}(C_2H_4O)_a\text{-}(C_3H_6O)_b\text{-}R'\text{-}N(H)\text{-}R\text{-}] \quad (K)$$

worin:

a eine ganze Zahl größer gleich 1, vorzugsweise im Bereich von 5 bis 200 und noch weiter bevorzugt von 5 bis 100 ist,
b eine ganze Zahl im Bereich von 0 bis 200, vorzugsweise im Bereich von 4 bis 200 und noch weiter bevorzugt von 5 bis 100 ist,
x eine ganze Zahl im Bereich von 1 bis 10.000 und noch weiter bevorzugt von 10 bis 5000 ist;
die Gruppen R gleich oder verschieden sind und für eine zweiwertige organische Gruppe stehen, die über eine Kohlenstoff-Silicium-Bindung an das benachbarte Siliciumatom und an ein Stickstoffatom gebunden ist, die Gruppen R' gleich oder verschieden sind und für eine zweiwertige organische Gruppe stehen, die über eine Kohlenstoff-Sauerstoff-Bindung an das benachbarte Sauerstoffatom und an ein Stickstoffatom gebunden ist.

3. Zusammensetzung nach dem vorhergehenden Anspruch, **dadurch gekennzeichnet, dass** sie mindestens ein Aminosilikon umfasst, das aus denjenigen der Formel (A) und (B) und Mischungen davon ausgewählt ist.

4. Zusammensetzung nach einem der Ansprüche 2 und 3, **dadurch gekennzeichnet, dass** das Silikonöl ein Polydimethylsiloxan mit Aminoethyliminoisobutylgruppen umfasst.

5. Zusammensetzung nach einem der vorhergehenden Ansprüche, **dadurch gekennzeichnet, dass** sie mindestens 0,01 Gew.-% Öl, bezogen auf das Gesamtgewicht der Zusammensetzung, umfasst.

6. Zusammensetzung nach dem vorhergehenden Anspruch, **dadurch gekennzeichnet, dass** sie 0,01 bis 20 Gew.-% Öl und vorzugsweise 0,1 bis 10 Gew.-% Öl, bezogen auf das Gesamtgewicht der Zusammensetzung, umfasst.

7. Zusammensetzung nach einem der vorhergehenden Ansprüche, **dadurch gekennzeichnet, dass** sie eine Mischung von mit 4 EO oxyethyleniertem Sorbitanmonolaurat und mit 20 EO oxyethyleniertem Sorbitanmonolaurat umfasst.

8. Zusammensetzung nach einem der vorhergehenden Ansprüche, **dadurch gekennzeichnet, dass** sie eine Gesamtmenge von mindestens 0,5 Gew.-% oxyethyleniertem Sorbitanester bzw. oxyethylenierten Sorbitanestern, bezogen auf das Gesamtgewicht der Zusammensetzung, umfasst.

9. Zusammensetzung nach dem vorhergehenden Anspruch, **dadurch gekennzeichnet, dass** sie eine Gesamtmenge an oxyethyleniertem Sorbitanester bzw. oxyethylenierten Sorbitanestern im Bereich von 0,5 bis 20 Gew.-%, weiter bevorzugt von 2 bis 15 Gew.-% und noch weiter bevorzugt von 4 bis 10 Gew.-%, bezogen auf das Gesamtgewicht der Zusammensetzung, umfasst.

10. Zusammensetzung nach einem der vorhergehenden Ansprüche, **dadurch gekennzeichnet, dass** sie mindestens ein anionisches Tensid umfasst, das aus Salzen der folgenden Typen ausgewählt ist: Alkylsulfaten, Alkylethersulfaten, Alkylamidoethersulfaten, Alkylarylpolyethersulfaten, Monoglyceridsulfaten, Alkylsulfonaten, Alkylamidsulfonaten, Alkylarylsulfonaten, $\alpha$-Olefinsulfonaten, Paraffinsulfonaten, Alkylsulfosuccinaten, Alkylethersulfosuccinaten, Alkylamidsulfosuccinaten, Alkylsulfoacetaten, Acylsarcosinaten und Acylglutamaten, wobei die Alkyl- und Acylgruppen aller dieser Verbindungen 6 bis 24 Kohlenstoffatome enthalten und die Arylgruppe vorzugsweise für eine Phenyl- oder Benzylgruppe steht.

11. Zusammensetzung nach einem der vorhergehenden Ansprüche, **dadurch gekennzeichnet, dass** sie mindestens ein anionisches Tensid umfasst, das ausgewählt ist aus $C_6$-$C_{24}$-Alkylmonoestern und Polyglycosiddicarbonsäuren wie Alkylglucosidcitraten, Alkylpolyglycosidtartraten, Alkylpolyglycosidsulfosuccinaten, Alkylsulfosuccinamaten, Acylisethionaten und N-Acyltauraten, wobei die Alkyl- bzw. Acylgruppe aller dieser Verbindungen 12 bis 20 Kohlenstoffatome enthält.

12. Zusammensetzung nach einem der vorhergehenden Ansprüche, **dadurch gekennzeichnet, dass** sie mindestens ein anionisches Tensid umfasst, das ausgewählt ist aus Acyllactylaten mit 8 bis 20 Kohlenstoffatomen in der Acylgruppe, Alkyl-D-galactosiduronsäuren, polyoxyalkylenierten ($C_6$-$C_{24}$-Alkyl)ethercarbonsäuren, polyoxyalkylenierten ($C_6$-$C_{24}$-Alkyl) ($C_6$-$C_{24}$-aryl)-ethercarbonsäuren und polyoxyalkylenierten ($C_6$-$C_{24}$-Alkyl)amidoethercarbonsäuren und Salzen dieser Verbindungen.

13. Zusammensetzung nach Anspruch 10, **dadurch gekennzeichnet, dass** sie mindestens ein anionisches Tensid umfasst, das ausgewählt ist aus Alkylsulfaten, Alkylethersulfaten und Mischungen davon, insbesondere in Form von Alkali- oder Erdalkalimetall-, Ammonium-, Aminoder Aminoalkohol-Salzen.

14. Zusammensetzung nach einem der vorhergehenden Ansprüche, **dadurch gekennzeichnet, dass** sie 0,5 bis 50 Gew.-% anionisches Tensid bzw. anionische Tenside, vorzugsweise 4 bis 20 Gew.-%, bezogen auf das Gesamtgewicht der Zusammensetzung, umfasst.

15. Zusammensetzung nach einem der vorhergehenden Ansprüche, **dadurch gekennzeichnet, dass** sie mindestens ein amphoteres oder zwitterionisches Tensid umfasst, das ausgewählt ist aus:

- Derivaten von sekundären oder tertiären aliphatischen Aminen, worin die aliphatische Gruppe eine lineare oder verzweigte Kette mit 8 bis 22 Kohlenstoffatomen und mindestens einer anionischen Gruppe, wie beispielsweise einer Carboxylat-, Sulfonat-, Sulfat-, Phosphat- oder Phosphonatgruppe, ist, und
- ($C_8$-$C_{20}$)-Alkylbetainen, Sulfobetainen, ($C_8$-$C_{20}$-Alkyl) amido ($C_6$-$C_8$-alkyl)betainen oder ($C_8$-$C_{20}$-Alkyl)amido($C_6$-$C_8$-alkyl)sulfobetainen.

16. Zusammensetzung nach einem der vorhergehenden Ansprüche, **dadurch gekennzeichnet, dass** sie 0,1 bis 10 Gew.-% amphoteres oder zwitterionisches Tensid bzw. amphotere oder zwitterionische Tenside, vorzugsweise 0,5 bis 8 Gew.-%, bezogen auf das Gesamtgewicht der Zusammensetzung, umfasst.

17. Zusammensetzung nach einem der Ansprüche 1 und/oder 2 bis 14, **dadurch gekennzeichnet, dass** das Gewichtsverhältnis zwischen der Menge an Öl einerseits und der Gesamtmenge an oxyethyleniertem Sorbitanester bzw. oxyethylenierten Sorbitanestern, anionischem Tensid bzw. anionischen Tensiden und amphoterem oder zwitterionischem Tensid bzw. amphoteren oder zwitterionischen Tensiden andererseits zwischen 0,01 und 0,2 und weiter bevorzugt zwischen 0,02 und 0,1 liegt.

18. Zusammensetzung nach einem der vorhergehenden Ansprüche, **dadurch gekennzeichnet, dass** das Gewichtsverhältnis zwischen der Konzentration an amphoterem oder zwitterionischem Tensid bzw. amphoteren oder zwitterionischen Tensiden einerseits und der Konzentration an anionischem Tensid bzw. anionischen Tensiden andererseits zwischen 0,1 und 0,5 liegt.

**19.** Zusammensetzung nach einem der vorhergehenden Ansprüche, **dadurch gekennzeichnet, dass** sie mindestens ein kationisches Tensid umfasst.

**20.** Zusammensetzung nach dem vorhergehenden Anspruch, **dadurch gekennzeichnet, dass** die Menge an kationischem Tensid bzw. kationischen Tensiden im Bereich von 0,01 bis 10 Gew.-%, vorzugsweise 0,05 bis 5 Gew.-%, bezogen auf das Gesamtgewicht der Zusammensetzung, liegt.

**21.** Zusammensetzung nach einem der vorhergehenden Ansprüche, **dadurch gekennzeichnet, dass** sie mindestens ein kationisches Polymer umfasst.

**22.** Zusammensetzung nach dem vorhergehenden Anspruch, **dadurch gekennzeichnet, dass** sie 0,01 bis 10 Gew.-% kationisches Polymer bzw. kationische Polymere, bezogen auf das Gesamtgewicht der Zusammensetzung, umfasst.

**23.** Zusammensetzung nach einem der vorhergehenden Ansprüche, **dadurch gekennzeichnet, dass** sie außerdem mindestens ein Antischuppenmittel, wie beispielsweise Piroctonolamin, Zinkpyrithion, Salicylsäure, Selendisulfid oder Mischungen davon, umfasst.

**24.** Zusammensetzung nach dem vorhergehenden Anspruch, **dadurch gekennzeichnet, dass** sie 0,001 bis 10 Gew.-% Antischuppenmittel, vorzugsweise 0,1 bis 5 Gew.-%, bezogen auf das Gesamtgewicht der Zusammensetzung, umfasst.

**25.** Zusammensetzung nach einem der vorhergehenden Ansprüche, **dadurch gekennzeichnet, dass** sie außerdem mindestens ein Additiv umfasst, das ausgewählt ist aus Mitteln zur Bekämpfung von Haarverlust, Oxidationsmitteln, Ceramiden und Pseudoceramiden, Vitaminen und Provitaminen, darunter Panthenol, pflanzlichen, tierischen, mineralischen oder synthetischen Ölen, Wachsen, Ceramiden und Pseudoceramiden, Lichtschutzmitteln, farbigen oder farblosen anorganischen oder organischen Pigmenten, Farbmitteln, Sequestriermitteln, Weichmachern, Lösungsvermittlern, Ansäuerungsmitteln, Alkalinisierungsmitteln, anorganischen oder organischen Verdickungsmitteln, Antioxidantien, Hydroxysäuren, Parfümen und Konservierungsmitteln.

**26.** Verwendung der Zusammensetzung nach einem der Ansprüche 1 bis 25 als Zusammensetzung zur kosmetischen Behandlung oder Pflege der Haare oder der Haut.

**27.** Verwendung der Zusammensetzung nach einem der Ansprüche 1 bis 25 als Shampoo.

**28.** Verfahren zur kosmetischen Behandlung von Keratinmaterialien, **dadurch gekennzeichnet, dass** es das Aufbringen einer wirksamen Menge einer Zusammensetzung nach einem der Ansprüche 1 bis 25 auf die Materialien umfasst.

## Claims

**1.** Cosmetic composition for washing keratin materials, consisting of a microemulsion comprising:

- water,
- sorbitan monolaurate oxyethylenated with 4 OE,
- at least one oil chosen from amino silicones,
- at least one anionic surfactant, and
- at least one amphoteric or zwitterionic surfactant.

**2.** Composition according to the preceding claim, **characterized in that** it comprises at least one amino silicone, chosen from:

a) the polysiloxanes corresponding to formula (A):

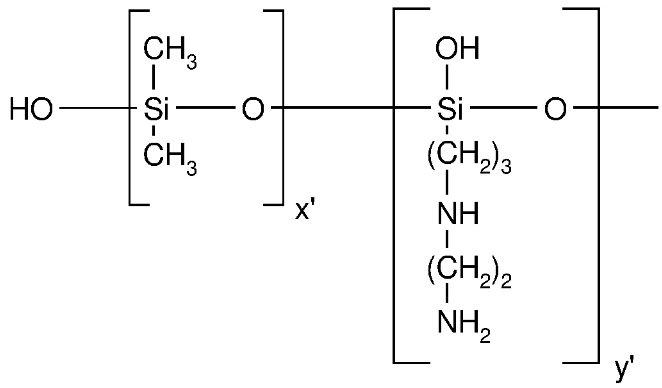

(A)

in which x' and y' are integers dependent on the molecular weight, which are generally such that the said weight-average molecular weight ranges from 5000 to 500 000;

b) the amino silicones corresponding to the formula:

$$R'_aG_{3-a}-Si(OSiG_2)_n-(OSiG_bR'_{2-b})_m-O-SiG_{3-a}-R'_a \qquad (B)$$

in which:

G, which may be identical or different, denote a hydrogen atom or a phenyl group, OH, $C_1$-$C_8$ alkyl or $C_1$-$C_8$ alkoxy,
a, which may be identical or different, denote the number 0 or an integer from 1 to 3,
b denotes 0 or 1,
m and n are numbers such that the sum (n + m) can especially range from 1 to 2000 and in particular from 50 to 150, n possibly denoting a number from 0 to 1999 and especially from 49 to 149, and m possibly denoting a number from 1 to 2000 and especially from 1 to 10;
R', which may be identical or different, denote a monovalent radical of formula -CqH2qL in which q is a number from 2 to 8 and L is an optionally quaternized amino group chosen from the groups:

- $NR''-Q-N(R'')_2$
- $N(R'')_2$
- $N^+(R'')_3$ A-
- $N^+H(R'')_2$ A-
- $N^+H_2$ (R'') A-
- $N(R'')-Q-N^+R''H_2$ A-
- $NR''-Q-N^+(R'')_2H$ A-
- $NR''-Q-N^+(R'')_3$ A-,

in which R'' may denote hydrogen, phenyl, benzyl, or a saturated monovalent hydrocarbon-based radical; Q denotes a linear or branched group of formula $C_rH_{2r}$, r being an integer ranging from 2 to 6 and preferably from 2 to 4; and A- represents a halide ion;

c) the amino silicones corresponding to formula (H) :

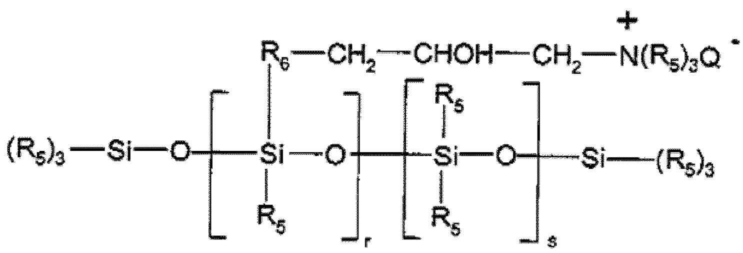

(H)

in which:

$R_5$ represents a monovalent hydrocarbon-based radical containing from 1 to 18 carbon atoms;
$R_6$ represents a divalent hydrocarbon-based radical linked to the Si via an SiC bond;
Q- is an anion such as a halide ion or an organic acid salt;
r represents a mean statistical value from 2 to 20;
s represents a mean statistical value from 20 to 200;

d) the quaternary ammonium silicones of formula (I) :

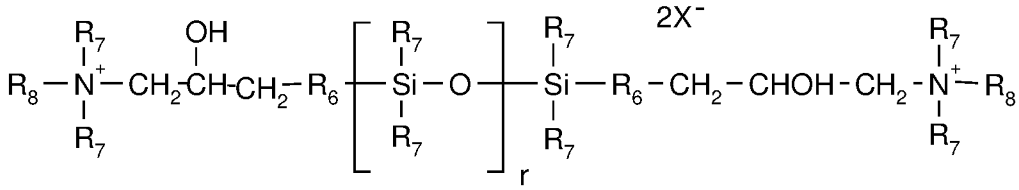

(I)

in which:

$R_7$, which may be identical or different, represent a monovalent hydrocarbon-based radical containing from 1 to 18 carbon atoms;
$R_6$ represents a divalent hydrocarbon-based radical linked to the Si via an SiC bond;
$R_8$, which may be identical or different, represent a hydrogen atom or a monovalent hydrocarbon-based radical containing from 1 to 18 carbon atoms;
X- is an anion such as a halide ion or an organic acid salt;
r represents a mean statistical value from 2 to 200;

e) the amino silicones of formula (J):

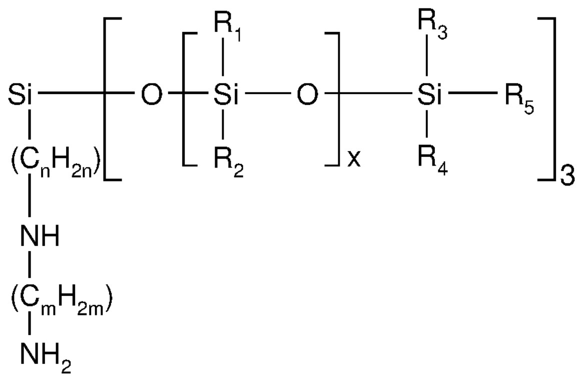

(J)

in which:

$R_1$, $R_2$, $R_3$ and $R_4$, which may be identical or different, denote a $C_1$-$C_4$ alkyl radical or a phenyl group,
$R_5$ denotes a $C_1$-$C_4$ alkyl radical or a hydroxyl group,
n is an integer ranging from 1 to 5,
m is an integer ranging from 1 to 5,
and in which x is chosen such that the amine number ranges from 0.01 to 1 meq/g;

f) the polyoxyalkylenated amino silicones of the type $(XY)_l$, X being a polysiloxane block and Y being a polyoxyalkylene block comprising at least one amine group, which may, preferably, consist of repeating units having the following general formula:

$$[SiMe_2\text{-}O\text{-}(SiMe_2O)_xSiMe_2\text{-}R\text{-}N(H)\text{-}R'\text{-}O\text{-}(C_2H_4O)_a\text{-}(C_3H_6O)_b\text{-}R'\text{-}N(H)\text{-}R\text{-}] \quad (K)$$

in which:

a is an integer greater than or equal to 1, preferably ranging from 5 to 200 and even more particularly from 5 to 100;
b is an integer ranging from 0 to 200, preferably ranging from 4 to 200 and even more particularly from 5 to 100;
x is an integer ranging from 1 to 10 000 and even more particularly from 10 to 5000;
R, which may be identical or different, represent a divalent organic group that is linked to the adjacent silicon atom via a carbon-silica bond and to a nitrogen atom,
R', which may be identical or different, represent a divalent organic group that is linked to the adjacent oxygen atom via a carbon-oxygen bond and to a nitrogen atom.

3. Composition according to the preceding claim, **characterized in that** it comprises at least one amino silicone chosen from those of formulae (A) and (B), and mixtures thereof.

4. Composition according to either of Claims 2 and 3, **characterized in that** the silicone oil comprises a polydimethylsiloxane containing aminoethyl iminoisobutyl groups.

5. Composition according to any one of the preceding claims, **characterized in that** it comprises at least 0.01% by weight of oil relative to the total weight of the composition.

6. Composition according to the preceding claim, **characterized in that** it comprises from 0.01% to 20% by weight of oil and preferably from 0.1% to 10% by weight of oil relative to the total weight of the composition.

7. Composition according to any one of the preceding claims, **characterized in that** it comprises a mixture of sorbitan monolaurate oxyethylenated with 4 OE and of sorbitan monolaurate oxyethylenated with 20 OE.

8. Composition according to any one of the preceding claims, **characterized in that** it comprises a total amount of at least 0.5% by weight of oxyethylenated sorbitan ester(s) relative to the total weight of the composition.

9. Composition according to the preceding claim, **characterized in that** it comprises a total amount of oxyethylenated sorbitan ester(s) ranging from 0.5% to 20% by weight, more preferentially from 2% to 15% by weight and even more preferentially from 4% to 10% by weight relative to the total weight of the composition.

10. Composition according to any one of the preceding claims, **characterized in that** it comprises at least one anionic surfactant chosen from salts of the following types: alkyl sulfates, alkyl ether sulfates, alkylamido ether sulfates, alkylarylpolyether sulfates, monoglyceride sulfates, alkyl sulfonates, alkylamide sulfonates, alkylaryl sulfonates, $\alpha$-olefin sulfonates, paraffin sulfonates, alkyl sulfosuccinates, alkyl ether sulfosuccinates, alkylamide sulfosuccinates, alkyl sulfoacetates, acyl sarcosinates and acyl glutamates, the alkyl and acyl groups of all these compounds containing from 6 to 24 carbon atoms, and the aryl group preferably denoting a phenyl or benzyl group.

11. Composition according to any one of the preceding claims, **characterized in that** it comprises at least one anionic surfactant chosen from $C_6$-$C_{24}$ alkyl monoesters of polyglycosidedicarboxylic acids, such as alkyl glucoside citrates, alkyl polyglycoside tartrates, alkyl polyglycoside sulfosuccinates, alkyl sulfosuccinamates, acyl isethionates and N-acyltaurates, the alkyl or acyl group of all these compounds containing from 12 to 20 carbon atoms.

12. Composition according to any one of the preceding claims, **characterized in that** it comprises at least one anionic surfactant chosen from acyl lactylates in which the acyl group contains from 8 to 20 carbon atoms, alkyl-D-galactoside uronic acids, polyoxyalkylenated ($C_6$-$C_{24}$ alkyl) ether carboxylic acids, polyoxyalkylenated ($C_6$-$C_{24}$ alkyl) ($C_6$-$C_{24}$ aryl) ether carboxylic acids, and polyoxyalkylenated ($C_6$-$C_{24}$ alkyl)amidoether carboxylic acids, and the salts of these compounds.

13. Composition according to Claim 10, **characterized in that** it comprises at least one anionic surfactant chosen from alkyl sulfates and alkyl ether sulfates, and mixtures thereof, in particular in the form of alkali metal or alkaline-earth metal, ammonium, amine or amino alcohol salts.

14. Composition according to any one of the preceding claims, **characterized in that** it comprises from 0.5% to 50% by weight and preferably from 4% to 20% by weight of anionic surfactant(s) relative to the total weight of the

composition.

**15.** Composition according to any one of the preceding claims, **characterized in that** it comprises at least one amphoteric or zwitterionic surfactant chosen from:

- secondary or tertiary aliphatic amine derivatives, in which the aliphatic group is a linear or branched chain containing from 8 to 22 carbon atoms and containing at least one anionic group, for instance a carboxylate, sulfonate, sulfate, phosphate or phosphonate group, and
- ($C_8$-$C_{20}$)alkylbetaines, sulfobetaines, ($C_8$-$C_{20}$ alkyl) amido ($C_6$-$C_8$ alkyl) betaines or ($C_8$-$C_{20}$ alkyl)amido($C_6$-$C_8$ alkyl)sulfobetaines.

**16.** Composition according to any one of the preceding claims, **characterized in that** it comprises from 0.1% to 10% by weight and preferably from 0.5% to 8% by weight of amphoteric or zwitterionic surfactants(s) relative to the total weight of the composition.

**17.** Composition according to any one of Claims 1 and/or 2 to 14, **characterized in that** the weight ratio between the amount of oil, on the one hand, and the total amount of oxyethylenated sorbitan ester(s), of anionic surfactant(s) and of amphoteric or zwitterionic surfactants(s), on the other hand, is between 0.01 and 0.2 and more preferentially between 0.02 and 0.1.

**18.** Composition according to any one of the preceding claims, **characterized in that** the weight ratio between the concentration of amphoteric or zwitterionic surfactant(s), on the one hand, and the concentration of anionic surfactant(s), on the other hand, is between 0.1 and 0.5.

**19.** Composition according to any one of the preceding claims, **characterized in that** it comprises at least one cationic surfactant.

**20.** Composition according to the preceding claim, **characterized in that** the amount of cationic surfactant (s) is in the range from 0.01% to 10% by weight and preferably from 0.05% to 5% by weight relative to the total weight of the composition.

**21.** Composition according to any one of the preceding claims, **characterized in that** it comprises at least one cationic polymer.

**22.** Composition according to the preceding claim, **characterized in that** it comprises from 0.01% to 10% by weight of cationic polymer(s) relative to the total weight of the composition.

**23.** Composition according to any one of the preceding claims, **characterized in that** it also comprises at least one antidandruff agent, for instance piroctone olamine, zinc pyrithione, salicylic acid or selenium disulfide, and mixtures thereof.

**24.** Composition according to the preceding claim, **characterized in that** it comprises from 0.001% to 10% by weight and preferably from 0.1% to 5% by weight of antidandruff agent(s) relative to the total weight of the composition.

**25.** Composition according to any one of the preceding claims, **characterized in that** it also comprises at least one additive chosen from hair-loss counteractants, oxidizing agents, ceramides and pseudoceramides, vitamins and provitamins including panthenol, plant, animal, mineral or synthetic oils, waxes, ceramides and pseudoceramides, sunscreens, coloured or uncoloured mineral or organic pigments, dyes, sequestrants, plasticizers, solubilizers, acidifying agents, basifying agents, mineral or organic thickeners, antioxidants, hydroxy acids, fragrances and preserving agents.

**26.** Use of the composition according to any one of Claims 1 to 25 as a cosmetic composition for treating or caring for the hair or the skin.

**27.** Use of the composition according to any one of Claims 1 to 25 as a shampoo.

**28.** Cosmetic process for treating keratin materials, **characterized in that** it comprises the application to the said materials of an effective amount of a composition according to any one of Claims 1 to 25.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2804020
- US 6153569 A
- EP 453238 A
- WO 0205758 A
- CA 1077849
- WO 03057185 A
- EP 95238 A
- US 4185087 A
- EP 0530974 A
- US 2528378 A
- US 2781354 A
- EP 0337354 A
- FR 2270846 A
- FR 2383660
- FR 2598611
- FR 2470596
- FR 2519863
- FR 2505348
- FR 2542997
- EP 080976 A
- FR 2077143
- FR 2393573
- FR 1492597
- US 4131576 A
- US 3589578 A
- US 4031307 A
- FR 2162025
- FR 2280361
- FR 2252840
- FR 2368508
- FR 1583363
- US 3227615 A
- US 2961347 A
- FR 2080759
- FR 2190406
- FR 2320330
- FR 2270846
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- EP 122324 A


### Littérature non-brevet citée dans la description


- **PAUL BECHER.** Dictionary of Colloid and Surface Science. Marcel Dekker Inc, 1990, 102
- CTFA. 1982
- CTFA. 1993
- **M.R. PORTER.** Handbook of Surfactants. Blackie & Son, 1991, 116-178